# EUROPÄISCHE PATENTSCHRIFT

(11) **EP 3 177 367 B1**
(45) Veröffentlichungstag und Bekanntmachung des Hinweises auf die Patenterteilung: **15.08.2018**
(21) Anmeldenummer: 15731347.9
(22) Anmeldetag: 25.06.2015
(51) Int. Cl.: A61Q 5/06, A61K 8/87, A61K 8/898

(54) **GLÄTTUNGSMITTEL, ENTHALTEND EINE POLYMERKOMBINATION AUS POLYURETHANEN UND AMODIMETHICONEN**
STRAIGHTENING AGENT CONTAINING A POLYMER COMBINATION OF POLYURETHANES AND AMODIMETHICONES
AGENT LISSANT CONTENANT UNE COMBINAISON POLYMÈRE COMPOSÉE DE POLYURÉTHANE ET D'AMODIMÉTHICONES

(30) Priorität: 06.08.2014 DE 102014215486
(43) Veröffentlichungstag der Anmeldung: 14.06.2017
(73) Patentinhaber: Henkel AG & Co. KGaA, 40589 Düsseldorf (DE)
(72) Erfinder: KNAPPE, Thorsten, 22869 Schenefeld (DE); MEISEL, Marie, 27386 Hemslingen (DE)
(86) Internationale Anmeldenummer: PCT/EP2015/064385
(87) Internationale Veröffentlichungsnummer: WO 2016/020108

(56) Entgegenhaltungen:
- EP-A1- 2 712 609
- DE-A1-102012 224 298
- JP-A- 2007 223 931
- "Polyurethanes for cosmetics Formulations for hair styling & treatment", , 23. April 2014 (2014-04-23), XP055204489, Gefunden im Internet: URL:http://biotechnologia.pl/uploads/biote chnologia/product/leaflet/368854/po__czone _pliki.pdf [gefunden am 2015-07-24]
- Anonymous: "Hair care dreams suddenly come true", , 17. März 2014 (2014-03-17), XP055204490, Gefunden im Internet: URL:http://webcache.googleusercontent.com/ search?q=cache:YbDcSyQ2L_cJ:www.materialsc ience.bayer.com/~/media/Country%2520Site/C hina/Documents/Press%2520Releases/English/ 20140317_hair%2520come%2520ture_EN.ashx+&c d=3&hl=en&ct=clnk&gl=de [gefunden am 2015-07-24]
- BETHANY JOHNSON: "Use of Amino-Polyether Block Copolymers in Hair Care Applications", IP.COM JOURNAL, IP.COM INC., WEST HENRIETTA, NY, US, 6. Oktober 2006 (2006-10-06), XP013116185, ISSN: 1533-0001

## Beschreibung

Die Erfindung betrifft das technische Gebiet der temporären Verformung keratinhaltiger Fasern, insbesondere menschlicher Haare.

Gegenstand der vorliegenden Erfindung ist ein kosmetisches Mittel zur temporären Verformung keratinischer Fasern, enthaltend in einem kosmetisch verträglichen Träger mindestens ein spezielles anionisches Polyurethanpolymer und mindestens ein spezielles siliconhaltiges kationisches Copolymer.

Ein weiterer Gegenstand der vorliegenden Erfindung ist ein Verfahren zur temporären Verformung keratinischer Fasern unter Einwirkung von Wärme, wobei ein kosmetisches Mittel, enthaltend mindestens ein spezielles anionisches Polyurethanpolymer und mindestens ein spezielles siliconhaltiges kationisches Copolymer, verwendet wird.

Schließlich ist ein weiterer Gegenstand der vorliegenden Erfindung die Verwendung eines kosmetischen Mittels, enthaltend mindestens ein spezielles anionisches Polyurethanpolymer und mindestens ein spezielles siliconhaltiges kationisches Copolymer, zur temporären Verformung keratinischer Fasern bei einer Temperatur von 30 bis 250 °C.

Keratinische Fasern, insbesondere menschliche Haare, werden heutzutage einer Vielzahl von Behandlungen unterzogen. Dabei spielen Behandlungen, welche zu einer permanenten oder temporären Formgebung der Haare dienen, um ansprechend aussehende Frisuren zu erhalten, eine wichtige Rolle. Aufgrund von aktuellen Modeströmungen gelten immer wieder Frisuren als chic, welche sich bei vielen Haartypen nur unter Verwendung bestimmter festigender Wirkstoffe aufbauen bzw. für einen längeren Zeitraum aufrechterhalten lassen. Als temporäre Formgebungen kommen beispielsweise Gestaltungen wie Lockung, Glättung, Toupierung oder auch Festigung in Frage.

Temporäre Formgebungen, welche einen guten Halt ergeben sollen, ohne das gesunde Aussehen der Haare, wie z.B. deren Glanz, zu beeinträchtigen, können beispielsweise durch Stylingmittel, wie Haarsprays, Haarwachse, Haargele, Haarfestiger, Fönwellen, Stylingsprays etc. erzielt werden.

Gleichfalls spielt im Bereich der temporären Formgebung der Haare die Haarverformung unter Wärmeeinwirkung, beispielsweise durch einen Fön, einen Lockenstab oder ein Glätteisen, eine wichtige Rolle. Zur Verbesserung von Stylingergebnissen wird oftmals vor einer Wärmeanwendung ein Stylingmittel, beispielsweise ein Haarfestiger oder ein Stylingspray, im Falle höherer Temperaturen auch ein sogenanntes thermisches Stylingspray, auf das Haar aufgetragen. Besonders im Friseurbereich finden sogenannte Glätteisen ("hot irons") immer mehr Verwendung. Glätteisen weisen zwei parallel angeordnete Metall- oder Keramikplatten auf, durch welche die Haare, nach Aufheizen der Platten, hindurchgezogen werden, indem das Glätteisen entlang einer Haarsträhne geführt wird. Handelsübliche Glätteisen lassen sich auf Temperaturen im Bereich von 150 bis 250 °C aufheizen. Ziel der Glätteisenanwendung ist es, gewelltes bis gelocktes Haar thermisch/physikalisch zu glätten.

Sollen Haare durch ein Glätteisen oder einen Fön geglättet werden, wird auf das Haar üblicherweise vorher ein thermisches Stylingsmittel, auch als Glätteisenmittel bezeichnet, aufgetragen. Das Mittel soll hierbei das Gleiten des Eisens und die Glättung des Haares unterstützen. Weierhin sollen derartige Mittel eine Haarschädigung aufgrund der bei der Glättung angewendeten hohen Temperaturen vorbeugen bzw. minimieren.

Mit den im Stand der Technik bekannten Stylingmitteln, insbesondere thermischen Stylingmitteln, lässt sich jedoch nicht immer eine zufriedenstellende Glättung erreichen. Weiterhin kann die Minimierung der Haarschädigung der im Stand der Technik bekannten Stylingmittel weitergehend verbessert werden (siehe z.B. Baycusan^{R} C1008 in "Polyurethanes for cosmetics; Formulations for hair styling & treatment", 23. April 2014, Bayer Material Science AG, Leverkusen). Der vorliegenden Erfindung lag die Aufgabe zugrunde, kosmetische Mittel zur temporären Verformung keratinischer Fasern bereitzustellen, welche eine verbessertes Frisurenergebnis in Form einer hohen Glättwirkung sowie eine verringerte Haarschädigung bzw. eine verbesserte Pflege, insbesondere eine verbesserte Nass- und/oder Trockenkämmbarkeit, aufweisen. Weiterhin sollen diese kosmetischen Mittel eine hohe Lagerstabilität besitzen.

Es wurde überraschenderweise gefunden, dass kosmetische Mittel zur temporären Verformung keratinischer Fasern eine hohe Glättwirkung sowie eine verringerte Haarschädigung gewährleisten, wenn diese Mittel eine Polymerkombination aus einem speziellen anionischen Polyurethanpolymer und einem speziellen siliconhaltigen kationischen Copolymer enthalten. Weiterhin treten bei Kombination dieser speziellen Polymere keine Inkompatibilitäten auf, so dass eine hohe Lagerstabilität der erfindungsgemäßen kosmetischen Mittel gewährleistet wird.

Gegenstand der vorliegenden Erfindung ist somit ein kosmetisches Mittel zur temporären Verformung keratinischer Fasern, enthaltend in einem kosmetisch verträglichen Träger
a) mindestens ein anionisches Polyurethanpolymer, umfassend mindestens eine Struktureinheit der Formel (I) und mindestens eine Struktureinheit der Formel (II) und mindestens eine Struktureinheit der Formel (III) und mindestens eine Struktureinheit der Formel (IV) und mindestens eine Struktureinheit der Formel (V) 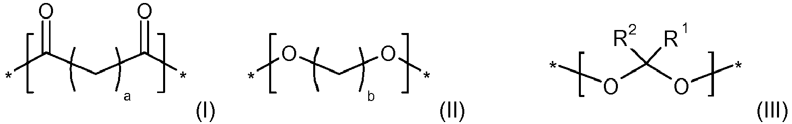 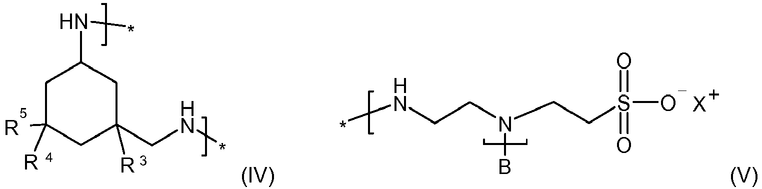 worin
   a und b, unabhängig voneinander, für ganze Zahlen von 1 bis 20 stehen,
   R¹, R², R³, R⁴ und R⁵, jeweils unabhängig voneinander, für ein Wasserstoffatom oder für eine C₁-C₄-Alkylgruppe stehen,
   B für ein Wasserstoffatom oder eine direkte Bindung zu einer weiteren Struktureinheit steht,
   X⁺ für ein physiologisch verträgliches Kation steht, und
b) mindestens ein siliconhaltiges kationisches Copolymer, umfassend mindestens eine Struktureinheit der Formel (VI) und mindestens eine Struktureinheit der Formel (VII) und mindestens eine Struktureinheit der Formel (VIII) 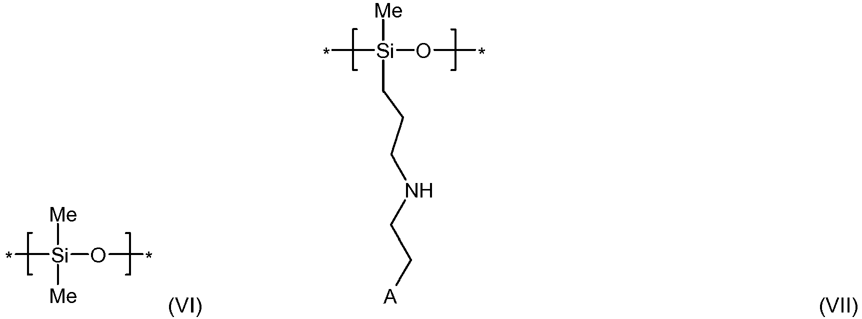 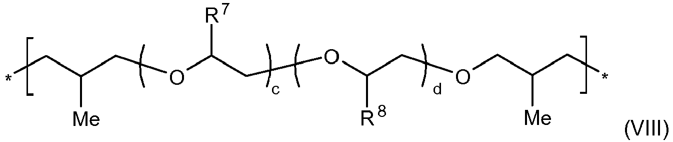 worin
   A für eine Hydroxylgruppe, eine Aminogruppe oder eine Thiolgruppe steht,
   R⁷ und R⁸, jeweils unabhängig voneinander, für ein Wasserstoffatom, eine Methylgruppe, eine Ethylgruppe, eine C₁-C₄-Aralkylgruppe, eine C₂-C₆-Alkenylgruppe oder eine C₂-C₆-Hydroxyalkylgruppe stehen, und
   c und d, jeweils unabhängig voneinander, für ganze Zahlen von 10 bis 55 stehen.

Das in den erfindungsgemäßen kosmetischen Mitteln eingesetzte anionische Polyurethanpolymer weist exzellente filmbildende Eigenschaften auf und gewährleistet somit einen hohen Frisurenhalt bzw. ein langanhaltendes Glättungsergebnis. Jedoch führt der alleinige Einsatz dieses anionischen Polyurethanpolymers nur zu einem unzureichenden Schutz vor Haarschädigungen gegenüber den bei der Glättung auftretenden hohen Temperaturen und somit nicht zu einer zufriedenstellenden Pflege der geglätteten Haare. Zur Erhöhung der Pflege während der Glättung ist daher der Einsatz von weiteren, insbesondere für ihre pflegende Wirkung im Stand der Technik bekannten kationischen Verbindungen erforderlich. Es hat sich jedoch gezeigt, dass das anionische Polyurethanpolymer aufgrund von Inkompatibilitäten nicht in Kombination mit den üblicherweise für die Erhöhung der Haarpflege verwendeten kationischen Pflegesubstanzen eingesetzt werden kann. Es war daher überraschend, dass das anionische Polyurethanpolymer mit Pflegesubstanzen in Form von speziellen siliconhaltigen kationischen Copolymeren kombiniert werden kann, ohne dass es zu Inkompatibilitäten zwischen diesen Polymeren kommt, welche in einer verminderten Lagerstabilität der erfindungsgemäßen kosmetischen Mittel oder in einem negativen Einfluss auf die hohe Glättungswirkung des anionischen Polyurethanpolymers und die hohe Hitzeschutzwirkung bzw. Pflegewirkung des siliconhaltigen kationischens Copolymers resultieren.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Weiterhin werden unter dem Begriff "Polyurethanpolymere" Polymere verstanden, bei welchen mindestens zwei Monomere, bevorzugt mindestens drei Monomere, über eine Urethan-Gruppierung -NH-CO-O- verknüpft sind. Erfindungsgemäß sind auch solche Polyurethanpolymere eingeschlossen, welche herstellungsbedingt harnstoffgruppenhaltige Wiederholungseinheiten -NH-C(O)-NH- aufweisen, wie sie insbesondere bei der Umsetzung von isocyanatterminierten Prepolymeren mit aminogruppenhaltigen Verbindungen gebildet werden. Erfindungsgemäß besonders bevorzugt sind Polyurethanpolymere, bei welchen alle Monomere über Urethan-Gruppierungen und/oder harnstoffgruppenhaltige Gruppierungen verknüpft sind. Derartige Polyurethanpolymere können beispielsweise durch Reaktion von zwei- oder mehrwertigen Alkoholen mit Diisocyanaten erhalten werden.

Darüber hinaus werden erfindungsgemäß unter dem Begriff "anionische Polyurethanpolymere" solche Polyurethanpolymere verstanden, welche in einem protischen Lösemittel bei Standardbedingungen mindestens eine Struktureinheit mit permanent anionischen Gruppen tragen, wobei die anionischen Gruppen durch Gegenionen unter Erhaltung der Elektroneutralität kompensiert werden müssen. Unter anionische Gruppen fallen erfindungsgemäß insbesondere Carboxyl- und Sulfonsäuregruppen.

Zudem werden im Rahmen der vorliegenden Erfindung unter dem Begriff "siliconhaltige kationische Copolymere" solche Copolymere verstanden, welche mindestens eine Gruppierung -Si(R)₂-O-enthalten und welche in einem protischen Lösemittel bei Standardbedingungen mindestens eine Struktureinheit mit einer kationischen Gruppen tragen, wobei die kationischen Gruppen ebenfalls durch Gegenionen unter Erhaltung der Elektroneutralität kompensiert werden müssen. Bevorzugt handelt es sich bei den siliconhaltigen kationischen Copolymeren um siliconhaltige Copolymere, welche kationische Gruppen in Form von protonierten Aminen enthalten.

Weiterhin sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die erfindungsgemäßen Mittel enthalten einen kosmetischen Träger. Erfindungsgemäß bevorzugt ist der kosmetische Träger wässrig, alkoholisch oder wässrig-alkoholisch.

Ein wässriger Träger enthält im Sinne der Erfindung mindestens 30 Gew.-%, insbesondere mindestens 50 Gew.-% Wasser, bezogen auf das Gesamtgewicht des kosmetischen Mittels.
Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Es kann erfindungsgemäß bevorzugt sein, wenn als zusätzliches Lösemittel des erfindungsgemäßen Mittels mindestens ein (C₂ bis C₆)-Alkylalkohol mit mindestens einer Hydroxygruppe eingesetzt wird. Im Rahmen einer besonders bevorzugten Ausführungsform des erfindungsgemäßen Mittels enthält das Mittel als zusätzliches Lösemittel mindestens einen Alkohol, welcher 2 bis 6 Kohlenstoffatome und 1 bis 3 Hydroxylgruppen aufweist.

Bevorzugt wird das von Wasser verschiedene Lösungsmittel ausgewählt aus mindestens einer Verbindung der Gruppe, welche gebildet wird aus Ethanol, Ethylenglykol, Isopropanol, 1,2-Propylenglykol, 1,3-Propylenglykol, Glyzerin, n-Butanol, 1,3-Butylenglykol. Ein ganz besonders bevorzugtes Lösemittel ist Ethanol.

Weitere, besonders bevorzugte Lösemittel sind Polyethylenglykol und/oder Polypropylenglykol.

Insbesondere der Zusatz von Polyethylenglykol und/oder Polypropylenglykol erhöht die Flexibilität des bei Anwendung des erfindungsgemäßen kosmetischen Mittels gebildeten Polymerfilms. Wird also ein flexibler Halt gewünscht, enthalten die erfindungsgemäßen kosmetischen Mittel vorzugsweise eine Gesamtmenge von 0,01 bis 30 Gew.-% Polyethylenglykol und/oder Polypropylenglykol, bezogen auf das Gesamtgewicht des kosmetischen Mittels.

Als ersten wesentlichen Bestandteil a) enthält das erfindungsgemäße kosmetische Mittel mindestens ein spezielles anionisches Polyurethanpolymer auf Basis der Struktureinheiten der Formeln (I) bis (V). In den Struktureinheiten der Formeln (III) und (IV) können die Reste R¹ bis R⁵ für (C₁ bis C₄)-Alkylgruppen stehen. Beispiele hierfür sind Methyl-, Ethyl-, Propyl-, Isopropyl-, Butyl-, Sec-Butyl-, Isobutyl- sowie tert-Butylgruppen.

Die in den erfindungsgemäßen kosmetischen Mitteln enthaltenen anionischen Polyurethanpolymere können durch Umsetzung eines isocyanatgruppenhaltigen Prepolymers (V1) mit mindestens einer aminogruppenhaltigen Verbindung (V2) erhalten werden.

Bevorzugt werden hierfür isocyanatgruppenhaltige Prepolymere (V1) verwendet, welche endständige Isocyanatgruppen aufweisen, d. h. derartige Polymere (V1) weisen Isocyanatgruppen an den Kettenenden, bevorzugt an sämtlichen Kettenenden, des Polymers auf. Weiterhin ist es bevorzugt, wenn isocyanatgruppenhaltige Prepolymere verwendet werden, welche durch Umsetzung von Isocyanaten bzw. Polyisocyanaten mit einem oder mehreren Polyolen, ausgewählt aus der Gruppe von Polyether-Polyolen, Polycarbonatpolyolen, Polyether-Polycarbonat-Polyolen und/oder Polyesterpolyolen, erhalten werden. Geeignete Prepolymere (V1) sind daher insbesondere die dem Fachmann an sich bekannten aliphatischen, aromatischen oder cycloaliphatischen Polyisocyanate mit einer NCO- Funktionalität von größer oder gleich 2.

Besonders bevorzugte Isocyanate sind Hexamethylendiisocyanat, Isophorondiisocyanat oder die isomeren Bis-(4,4'-isocyanatocyclohexyl)methane sowie Mischungen der vorgenannten Diisocyanate. Besonders bevorzugte aliphatische Polyesterpolyole sind Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol mit einem mittleren Molekulargewicht Mw von 600 bis 3000 g/mol. Weiterhin werden bevorzugt Polycarbonate, insbesondere Polydicarbonatdiole, mit mindestens einer Hydroxylgruppe und einem mittleren Molekulargewicht Mw von 600 bis 3000 g/mol eingesetzt, welche beispielsweise durch Reaktion von Kohlensäurederivaten, wie Diphenylcarbonat, Dimethylcarbonat oder Phosgen, mit Polyolen, bevorzugt Diolen, erhältlich sind. Ebenfalls bevorzugt können Polyetherpolyole, wie Polytetramethylenglykolpolyether und Polycarbonat-Polyole bzw. deren Mischungen eingesetzt werden. Die Bestimmung des mittleren Molekulargewichts M_{w} kann beispielsweise mittels Gelpermeationschromatographie (GPC) mit Polystyrol als internem Standard, wie in der deutschen Offenlegungsschrift DE1999614603 beschrieben, erfolgen.

Die zur Herstellung der erfindungsgemäß eingesetzten anionischen Polyurethanpolymere verwendeten aminogruppenhaltigen Verbindungen (V2) werden bevorzugt aus primären und/oder sekundären Aminen und/oder Diaminen ausgewählt. Insbesondere umfassen die aminogruppenhaltigen Verbindungen (V2) mindestens ein Diamin. Die aminogruppenhaltigen Verbindungen (V2) werden bevorzugt aus aminogruppenhaltigen Verbindungen, welche ionische oder ionogene (ionenbildende) Gruppen aufweisen, und aminogruppenhaltigen Verbindungen, welche keine ionischen oder ionogenen Gruppen aufweisen, ausgewählt. Geeignete aminogruppenhaltige Verbindungen (V2), welche keine ionischen oder ionogenen Gruppen aufweisen, sind bevorzugt ausgewählt aus 1,2-Ethylendiamin, Bis(4-aminocyclohexyl)methan, 1,4-Diaminobutan, Isophorondiamin, Ethanolamin, Diethanolamin und Diethylentriamin. Aminogruppenhaltige Verbindungen (V2), welche ionische und/oder ionogene Gruppen enthalten, weisen bevorzugt Sulfonat- bzw. Sulfonsäuregruppen, besonders bevorzugt Natriumsulfonatgruppen, auf. Aminogruppenhaltige Verbindungen (V2), welche ionische oder ionogene Gruppen aufweisen, sind bevorzugt ausgewählt aus Salzen von 2-(2-Aminoethylamino)ethansulfonsäure, Ethylendiaminpropyl- oder -butylsulfonsäure, 1,2- oder 1,3-Propylendiamin-ß-ethylsulfonsäure sowie Taurin.

Gemäß einer ersten Ausführungsform der vorliegenden Erfindung steht in der Struktureinheit der Formel (I) a für ganze Zahlen von 1 bis 10, vorzugsweise von 2 bis 8, insbesondere von 2 bis 6 und steht in der Struktureinheit der Formel (II) b für ganze Zahlen von 1 bis 12, vorzugsweise von 2 bis 10, insbesondere von 4 bis 8.

Im Rahmen der vorliegenden Erfindung kann es darüber hinaus vorgesehen sein, dass in den Struktureinheiten der Formeln (III) und (IV) die Reste R¹, R², R³, R⁴ und R⁵, jeweils unabhängig voneinander, für Wasserstoff oder für eine Methylgruppe, insbesondere für eine Methylgruppe, stehen.

Es ist erfindungsgemäß bevorzugt, wenn in der Struktureinheit der Formel (I) X⁺ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht. Kationische organische Verbindungen können beispielsweise durch Protonierung primärer, sekundärer oder tertiärer organischer Amine mit einer Säure oder durch permanente Quaternisierung besagter organischer Amine erhalten werden. Beispiele für im Rahmen der vorliegenden Erfindung geeignete kationische organische Ammoniumverbindungen sind beispielsweise 2-Ammonioethanol und 2-Trimethylammonioethanol.

Gemäß einer besonders bevorzugten Ausführungsform der vorliegenden Erfindung enthält das erfindungsgemäße kosmetische Mittel mindestens ein anionisches Polyurethanpolymer a), welches mindestens eine Struktureinheit der Formel (la) und mindestens eine Struktureinheit der Formel (IIa) und mindestens eine Struktureinheit der Formel (lila) und mindestens eine Struktureinheit der Formel (IVa) und mindestens eine Struktureinheit der Formel (Va) aufweist 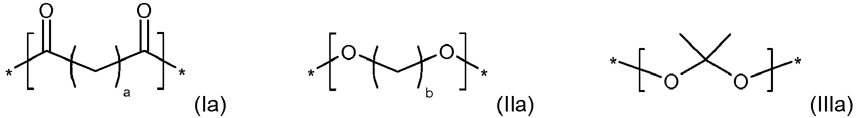 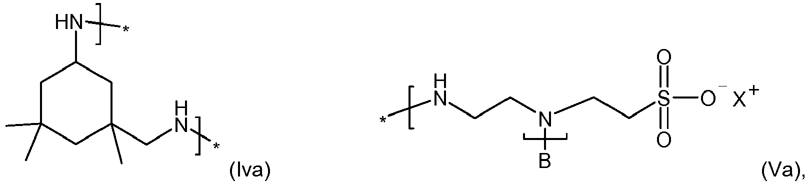 worin
a für ganze Zahlen von 2 bis 6 steht,
b für ganze Zahle von 4 bis 8 steht, und
X⁺ für ein physiologisch verträgliches Kation, insbesondere Natrium, steht. Der Einsatz der zuvor genannten speziellen anionischen Polyurethanpolymere in den erfindungsgemäßen kosmetischen Mitteln führt zu einem hervorragenden Frisurenhalt, insbesondere zu einer hervorragenden Glättungswirkung. Weiterhin wird der hervorragender Frisurenhalt über einen langen Zeitraum gewährleistet.
Ein im Rahmen dieser Ausführungsform ganz besonders bevorzugtes anionisches Polyurethanpolymer ist das unter der INCI-Bezeichnung Polyurethan-48 bekanntes Polymer. Dieses Polymer enthält Adipinsäure (a in Struktureinheit der Formel (la) steht für die ganze Zahl 4) und 1,6-Hexandiol (b gemäß Struktureinheit der Formel (IIa) steht für die ganze Zahl 6).

Erfindungsgemäß bevorzugt eingesetzte anionische Polyurethanpolymere weisen eine bestimmte Glasübergangstemperatur T_{g} auf. Daher ist es im Rahmen der vorliegenden Erfindung bevorzugt, wenn das mindestens eine anionische Polyurethanpolymer a) eine Glasübergangstemperatur T_{g} von -70 °C bis -20 °C, vorzugsweise von -65 °C bis -25 °C, bevorzugt von -60 °C bis -30 °C, insbesondere von -50 °C bis -40 °C, aufweist. Die Glasübergangstemperaturen werden mittels dynamischer Differenzkalorimetrie (DSC) bei einer Heizrate von 10 K/min, einer Starttemperatur von mindestens 30 °C unterhalb der Glasübergangstemperatur und einer Endtemperatur von mindestens 30 °C oberhalb der Glasübergangstemperatur bestimmt. Die Glasübergangstemperatur T_{g} ergibt sich als Mittelpunkttemperatur nach der Tangentenmethode.

Bevorzugte erfindungsgemäße kosmetische Mittel enthalten das mindestens eine anionische Polyurethanpolymer a) in einer Gesamtmenge von 0,05 bis 5 Gew.-%, vorzugsweise von 0,08 bis 3 Gew.-%, bevorzugt von 0,1 bis 2 Gew.-%, insbesondere von 0,2 bis 1,5 Gew.-%, bezogen auf das Gesamtgewicht des kosmetischen Mittels. Die Verwendung der zuvor genannten Mengen des speziellen anionischen Polyurethanpolymers a) resultiert zum einen in einer hervorragenden Glättungswirkung der erfindungsgemäßen kosmetischen Mittel. Anderseits stellt der Einsatz dieser Mengen sicher, dass keine negativen Wechselwirkungen mit anderen Inhaltstoffen, insbesondere mit dem siliconhaltigen kationischen Copolymer auftreten, welche die Lagerstabilität sowie die Glättungswirkung der erfindungsgemäßen kosmetischen Mittel negativ beeinflussen können.

Als zweiten wesentlichen Bestandteil b) enthält das erfindungsgemäße kosmetische Mittel mindestens ein spezielles siliconhaltiges kationisches Copolymer auf Basis der Struktureinheiten der Formeln (VI) bis (VIII). Der Einsatz dieses siliconhaltigen kationischen Copolymers resultiert in einer hohen Pflegewirkung bzw. in einem hohen Schutz vor Haarschädigungen der mit den erfindungsgemäßen kosmetischen Mittel verformten, insbesondere geglätteten, keratinischen Fasern.

Im Hinblick auf die Pflegewirkung, insbesondere die Verminderung der Haarschädigung, der erfindungsgemäßen kosmetischen Mittel hat es sich als vorteilhaft erwiesen, wenn in der Struktureinheit der Formel (VII) der Rest A für eine Aminogruppe steht.

Besonders gute Ergebnisse werden im Rahmen der vorliegenden Erfindung erhalten, wenn in der Struktureinheit der Formel (VIII) die Reste R⁷ und R⁸, jeweils unabhängig voneinander, für ein Wasserstoffatom oder eine Methylgruppe, bevorzugt R⁷ für Wasserstoff und R⁸ für eine Methylgruppe, stehen.

Weiterhin ist es im Rahmen der vorliegenden Erfindung besonders bevorzugt, wenn in der Struktureinheit der Formel (VIII) c für ganze Zahlen von 15 bis 30, insbesondere von 15 bis 25, steht und d für ganze Zahlen von 20 bis 45, bevorzugt von 25 bis 40, steht.

Im Rahmen der vorliegenden Erfindung ist es daher ganz besonders bevorzugt, wenn das kosmetische Mittel mindestens ein kationisches siliconhaltiges Copolymer enthält, welches mindestens eine mindestens eine Struktureinheit der Formel (VIa) und mindestens eine Struktureinheit der Formel (VIIa) und mindestens eine Struktureinheit der Formel (VIIIa) enthält 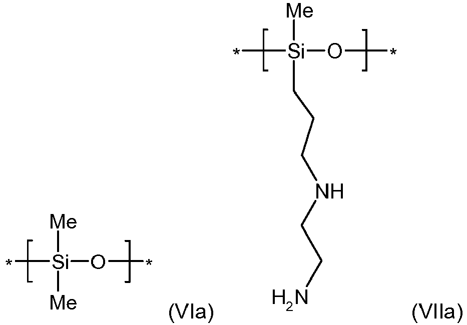 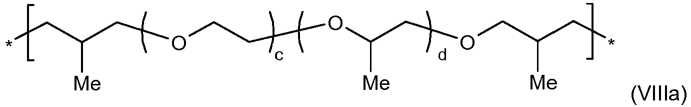 worin
c für ganze Zahlen von 15 bis 25 steht, und
d für ganze Zahlen von 25 bis 40 steht. Der Einsatz der zuvor genannten speziellen siliconhaltigen kationischen Copolymere in den erfindungsgemäßen kosmetischen Mitteln führt zu einem hervorragenden Schutz vor Haarschädigungen, welche aufgrund der während der Glättung verwendeten hohen Temperaturen auftreten können, und zu einer hervorragenden Pflege, insbesondere einer verbesserten Nass- und/oder Trockenkämmbarkeit. Darüber hinaus weisen diese speziellen siliconhaltigen kationischen Copolymere keine negativen Wechselwirkungen mit den speziellen anionischen Polyurethanpolymeren auf, so dass eine hohe Lagerstabilität sowie ein verbesserter Schutz vor Haarschädigungen und eine verbesserte Pflege der erfindungsgemäßen kosmetischen Mittel gewährleistet wird.

Ein im Rahmen dieser Ausführungsform ganz besonders bevorzugtes kationisches siliconhaltiges Copolymer ist ein Polymer mit der INCI-Bezeichnung Bis-Isobutyl PEG/PPG-20/35/Amodimethicone Copolymer. Dieses Polymer enthält etwa 20 Mol Ethylenoxid (c in Struktureinheit der Formel (Vlla) steht für die ganze Zahl 20) und etwa 35 Mol Propylenoxid (d gemäß Struktureinheit der Formel (VIIIa) steht für die ganze Zahl 35).

Bevorzugte erfindungsgemäße kosmetische Mittel enthalten das mindestens eine siliconhaltiges Copolymer b) in einer Gesamtmenge von 0,01 bis 2 Gew.-%, vorzugsweise von 0,02 bis 1,5 Gew.-%, bevorzugt 0,03 bis 1 Gew.-%, insbesondere 0,05 bis 0,5 Gew.-%, bezogen auf das Gesamtgewicht des kosmetischen Mittels. Die Verwendung der zuvor genannten Mengen des speziellen siliconhaltigen kationischen Copolymers b) resultiert zum einen in einer hervorragenden Pflegewirkung, insbesondere einer verbesserten Nass- und/oder Trockenkämmbarkeit, bzw. einem hervorragenden Schutz vor Haarschädigungen der erfindungsgemäßen kosmetischen Mittel. Anderseits stellt der Einsatz dieser Mengen sicher, dass keine negativen Wechselwirkungen mit anderen Inhaltstoffen, insbesondere mit dem anionischen Polyurethanpolymer auftreten, welche die Lagerstabilität sowie die Glättungswirkung und den Schutz vor Haarschädigungen der erfindungsgemäßen kosmetischen Mittel negativ beeinflussen können.

Erfindungsgemäße kosmetische Mittel, enthaltend in einem kosmetisch verträglichen Träger
a) mindestens ein anionisches Polyurethanpolymer, welches mindestens eine Struktureinheit der Formel (la) und mindestens eine Struktureinheit der Formel (IIa) und mindestens eine Struktureinheit der Formel (lila) und mindestens eine Struktureinheit der Formel (IVa) und mindestens eine Struktureinheit der Formel (Va) aufweist 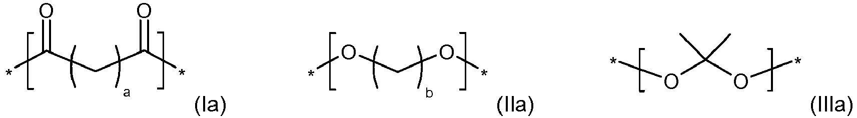 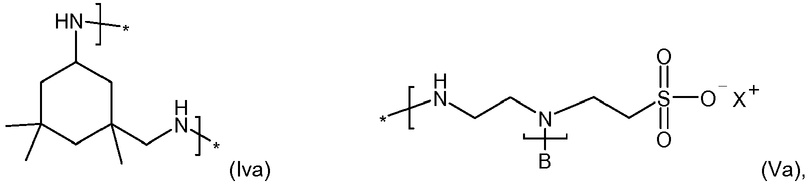 worin
   a für ganze Zahlen von 2 bis 6 steht,
   b für ganze Zahlen von 4 bis 8 steht, und
   X⁺ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht, und
b) mindestens ein kationisches siliconhaltiges Copolymer, welches mindestens eine mindestens eine Struktureinheit der Formel (VIa) und mindestens eine Struktureinheit der Formel (Vlla) und mindestens eine Struktureinheit der Formel (VIIIa) enthält 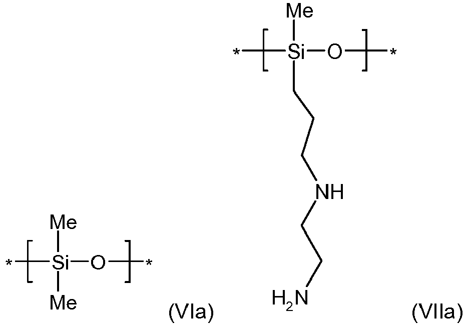 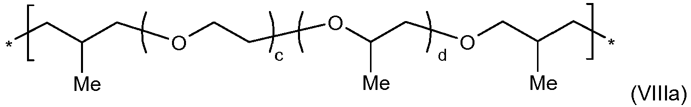 worin
   c für ganze Zahlen von 15 bis 25 steht, und
   d für ganze Zahlen von 25 bis 40 steht,
   sind im Rahmen der vorliegenden Erfindung besonders bevorzugt. Der Einsatz der zuvor genannten speziellen Polymerkombination ist besonders vorteilhaft im Hinblick auf einen hervorragenden Frisurenhalt, insbesondere in Form einer hervorragenden Glättungswirkung, sowie einen hervorragenden Schutz vor Haarschädigungen bzw. einer hevorragenden Pflege, insbesondere Nass- und/oder Trockenkämmbarkeit. Darüber hinaus treten bei Kombination der zuvor genannten speziellen Polymere keine negativen Wechselwirkungen auf, welche zu einer verminderten Lagerstabilität sowie zu einer verminderten Glättungswirkung und Pflegewirkung der erfindungsgemäßen kosmetischen Mittel führen.

Besonders gute Ergebnisse im Rahmen der vorliegenden Erfindung werden erhalten, wenn das kosmetische Mittel ein Gewichtsverhältnis der Gesamtmenge des mindestens einen anionischen Polyurethanpolymers a) zu der Gesamtmenge des mindestens einen siliconhaltigen Copolymers b) von 300 : 1 bis 1 : 40, vorzugsweise von 100 : 1 bis 1 : 20, bevorzugt von 40 : 1 bis 1 : 5, weiter bevorzugt von 20 : 1 bis 1 : 1, insbesondere von 10 : 1 bis 2 : 1, aufweist. Bei den zuvor genannten Gewichtsverhältnissen treten keine negativen Wechselwirkungen zwischen dem speziellen anionischen Polyurethanpolymer und dem speziellen siliconhaltigen kationischen Copolymer auf, so dass die erfindungsgemäßen kosmetischen Mittel sowohl die hervorragenden Glättungseigenschaften des anionischen Polyurethanpolymers als auch die hevorragenden Pflegeeigenschaften bzw. die hervorragenden Schutzeigenschaften des siliconhaltigen kationischen Copolymers besitzen. Weiterhin weisen erfindungsgemäße kosmetische Mittel mit den zuvor genannten Gewichtsverhältnissen wegen der Abwesenheit ungünstiger negativer Wechselwirkungen zwischen den speziellen Polymeren eine hohe Lagerstabilität auf.

Im Rahmen der vorliegenden Erfindung ist es bevorzugt, wenn das kosmetische Mittel einen pH-Wert von pH 3,0 bis pH 7,0, vorzugsweise von pH 3,5 bis pH 6,0, insbesondere von pH 4,0 bis pH 5,5, aufweist. Derartige pH-Werte stellen sicher, dass das siliconhaltige Copolymer als kationische Verbindung vorliegt und eine hohe Affinität zu den keratinischen Fasern aufweist. Die hohe Affinität zu den keratinischen Fasern gewährleistet eine gleichmäßige Bedeckung dieser Fasern und somit einen effektiven Schutz dieser Fasern von einer Schädigung durch die während der Glättung angewendeten hohen Temperaturen. Die Einstellung des pH-Wertes kann hierbei unter Verwendung von in Stylingmitteln üblicherweise eingesetzten Säuren und Basen, bevorzugt organischen Säuren, wie beispielsweise Milchsäure und/oder Zitronensäure, erfolgen.

Die Glättungswirkung sowie der Schutz keratinischer Fasern vor Haarschädigungen kann weitergehend gesteigert werden, wenn das kosmetische Mittel zusätzlich ein anionisches Polymer, umfassend mindestens eine Struktureinheit der Formel (IX) und mindestens eine Struktureinheit der Formel (X) 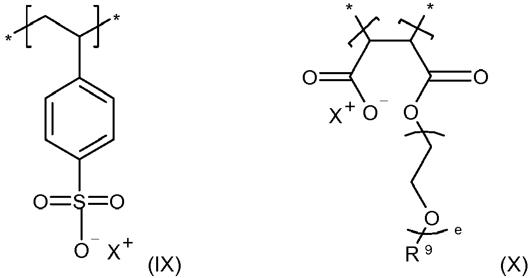 worin
R⁹ für eine lineare oder verzweigte, gesättigte oder ungesättigte C₁₀-C₄₀-Alkylgruppe oder eine C₁₀-C₄₀-Hydroxyalkylgruppe, insbesondere für eine lineare oder verzweigte, gesättigte oder ungesättigte C₁₄-C₃₆-Alkylgruppe, steht,
e für ganze Zahlen zwischen 20 und 70, vorzugsweise 30 und 60, insbesondere 35 und 45, steht, und
X⁺ für ein physiologisch verträgliches Kation steht,
enthält.

Der Langzeithalt des erfindungsgemäßen kosmetischen Mittels kann weitergehend gesteigert werden, wenn das kosmetische Mittel zusätzlich mindestens ein filmbildendes und/oder festigendes Polymer enthält, welches von dem anionischen Polyurethanpolymer a) verschieden ist. Filmbildende bzw. festigende Polymere tragen durch Filmbildung zum Halt der aufgeprägten Form des Faserkollektivs, z.B. der Gesamtfrisur, bei. Die Filmbildung kann dabei durchaus punktuell sein und nur einige Fasern miteinander verbinden. Unter filmbildenden bzw. festigenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, welche eine ausreichende Löslichkeit in Wasser oder Wasser/Alkohol-Gemischen besitzen, um in den erfindungsgemäßen kosmetischen Mitteln in vollständig gelöster Form vorzuliegen. Die filmbildenden Polymere können synthetischen oder natürlichen Ursprungs sein. Unter filmbildenden Polymeren werden weiterhin auch solche Polymere verstanden, welche bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Die zusätzlichen festigenden Polymere werden bevorzugt ausgewählt unter nichtionischen festigenden Polymeren, amphoteren festigenden Polymeren, kationischen festigenden Polymeren und anionischen festigenden Polymeren, besonders bevorzugt unter nichtionischen festigenden Polymeren, anionischen festigenden Polymeren, amphoteren festigenden Polymeren.

Das erfindungsgemäße Mittel enthält bevorzugt zusätzlich mindestens ein festigendes nichtionisches Polymer, enthaltend mindestens eine Struktureinheit aus der Gruppe der Struktureinheiten der Formeln (P1) bis (P6) 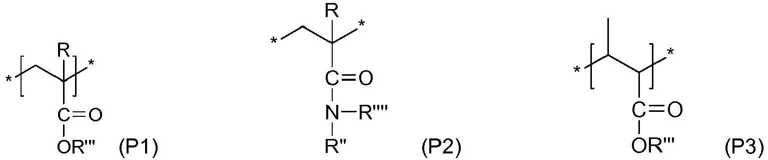 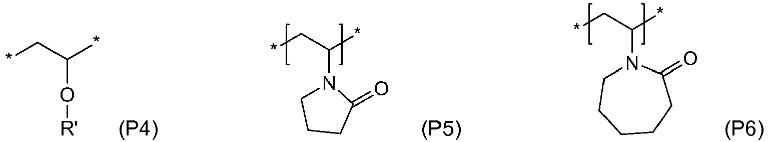 worin
R für ein Wasserstoffatom oder eine Methylgruppe steht,
R' für ein Wasserstoffatom oder eine (C₁ bis C₄)-Acylgruppe steht,
R" und R"", unabhängig voneinander, für eine (C₁ bis C₇)-Alkylgruppe oder ein Wasserstoffatom stehen
R'" für eine lineare oder verzweigte (C₁ bis C₄)-Alkylgruppe oder eine (C₂ bis C₄)-Hydroxyalkylgruppe steht.

Bevorzugte, nichtionische festigende Polymere, welche mindestens eine der zuvor genannten Struktureinheiten (P1) bis (P6) enthalten, sind Homo-oder Copolymere, welche aus mindestens einem der folgenden Monomere aufgebaut sind: N-Vinylpyrrolidon, N-Vinylcaprolactam, Vinylester (wie z.B. Vinylacetat, Vinylalkohol), Acrylamid, Methacrylamid, Alkyl- und Dialkylacrylamid (insbesondere N-Methyl- und N,N-Dimethylacrylamid), Alkyl- und Dialkylmethacrylamid (insbesondere N-Methyl- und N,N-Dimethylmethacrylamid), Alkylacrylat, Alkylmethacrylat, wobei jeweils die Alkylgruppen dieser Monomere aus (C₁ bis C₃)-Alkylgruppen ausgewählt werden.

Besonders geeignete nichtionische Polymere auf Basis ethylenisch ungesättigter Monomere enthalten mindestens eine der nachfolgenden Struktureinheiten 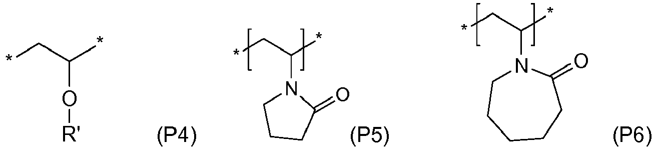 worin R' für ein Wasserstoffatom oder eine (C₁- bis C₃₀)-Acylgruppe, insbesondere für ein Wasserstoffatom oder eine Acetylgruppe, steht.

Geeignete Polymere, enthaltend mindestens eine der Struktureinheiten (P4) bis (P6) sind insbesondere Homopolymere des Vinylcaprolactams oder des Vinylpyrrolidons (wie beispielsweise Luviskol® K 90 oder Luviskol® K 85 der Firma BASF SE), Copolymerisate aus Vinylpyrrolidon und Vinylacetat mit einem Molverhältnis der aus dem Monomer N-Vinylpyrrolidon enthaltenen Struktureinheiten zu der aus dem Monomer Vinylacetat enthaltenen Struktureinheiten des Polymers im Bereich von 20 zu 80 bis 80 zu 20, insbesondere von 30 zu 70 bis 60 zu 40 (beispielsweise vertrieben unter dem Warenzeichen Luviskol® VA 37, Luviskol® VA 55, Luviskol® VA 64 und Luviskol® VA 73 von der Firme BASF SE), Terpolymere aus Vinylpyrrolidon, Vinylacetat und Vinylpropionat, Polyacrylamide (wie beispielsweise Akypomine® P 191 von der Firma CHEM-Y), Polyvinylalkohole (die beispielsweise unter den Handelsbezeichnungen Elvanol® von Du Pont oder Vinol® 523/540 von der Firma Air Products vertrieben werden), Terpolymere aus Vinylpyrrolidon, Methacrylamid und Vinylimidazol (wie beispielsweise Luviset® Clear der Firma BASF SE).

Erfindungsgemäße kosmetische Mittel, welche als festigendes nichtionisches Polymer mindestens ein Polymer enthalten, welches ausgewählt ist aus der Gruppe von
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
sind ganz besonders bevorzugt.

Im Rahmen einer weiteren Ausführungsform enthält das erfindungsgemäße kosmetische Mittel als zusätzliches festigendes Polymer mindestens ein festigendes anionisches Polymer.

Bevorzugte festigende anionische Polymere enthalten mindestens eine Struktureinheit der Formel (P7) und mindestens eine Struktureinheit der Formel (P8), 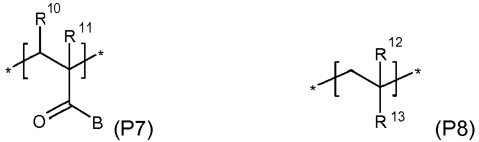 worin
R¹⁰ und R¹¹, unabhängig voneinander, für ein Wasserstoffatom oder eine Methylgruppe stehen, mit der Maßgabe, dass R¹⁰ und R¹¹ nicht gleichzeitig für eine Methylgruppe stehen,
R¹² für ein Wasserstoffatom oder eine Methylgruppe steht,
R¹³ für eine Carbamoyl-Gruppe, eine lineare oder verzweigte (C₄ bis C₁₂)-Alkylaminocarbonylgruppe, eine lineare oder verzweigte (C₄ bis C₁₂)-Alkyloxycarbonylgruppe, eine lineare oder verzweigte (C₂ bis C₁₂)-Acyloxygruppe, eine (C₂ bis C₄)-Hydroxyalkylcarbonylgruppe oder eine Phenylgruppe steht, und
B für eine Hydroxygruppe oder einen organischen Rest mit mindestens einer Sulfonsäuregruppe steht, welcher über ein Sauerstoffatom oder eine Gruppe NH an die Struktureinheit bindet.

In diesem Zusammenhang kann es bevorzugt sein, wenn das anionische festigende Polymer mindestens eine Struktureinheit der Formel (P7) enthält, welche ausgewählt wird aus mindestens einer Struktureinheit der Formeln (P7-I) bis (P7-V)

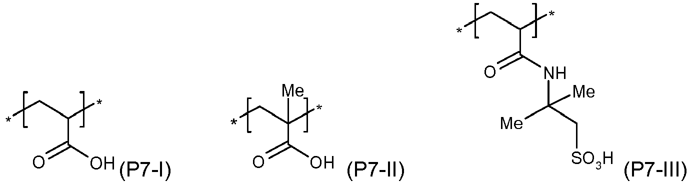

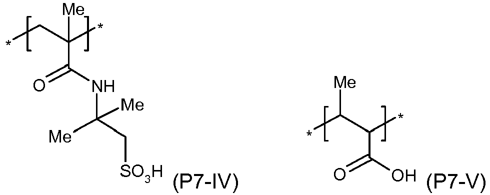

Es ist erfindungsgemäß besonders bevorzugt, wenn das anionische festigende Polymer mindestens eine Struktureinheit der Formel (P8) enthält, welche ausgewählt wird aus mindestens einer Struktureinheit der Formeln (P8-I) bis (P8-VII) 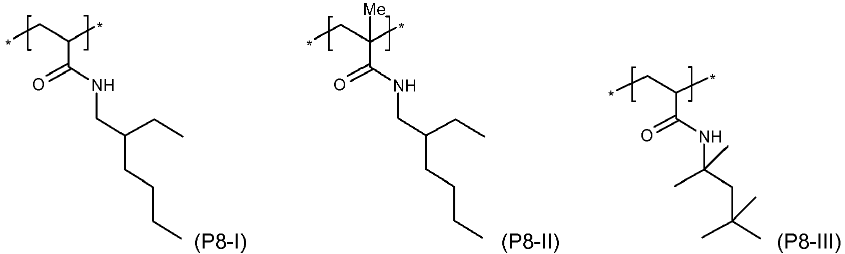 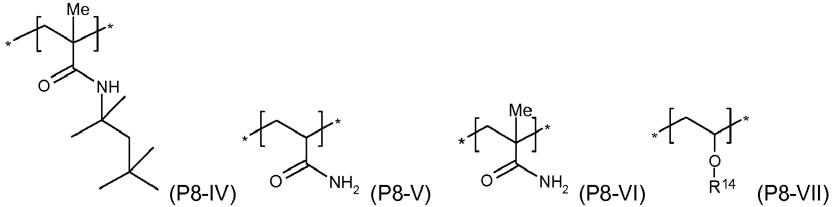 worin
R¹⁴ für eine (C₂ bis C₁₂)-Acylgruppe, insbesondere für Acetyl oder Neodecanoyl, steht.

Außerdem ist es bevorzugt, wenn das anionische festigende Polymer neben obigen Struktureinheiten der Formeln (P7) und (P8) zusätzlich mindestens eine Struktureinheit der Formel (P9) enthält 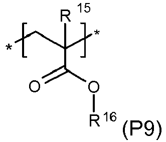 worin
R¹⁵ für ein Wasserstoffatom oder eine Methylgruppe steht, und
R¹⁶ für eine (C₁ bis C₃)-Alkylgruppe, insbesondere eine Methylgruppe oder eine Ethylgruppe, steht.

Im Allgemeinen ist es besonders bevorzugt, wenn in allen vorherigen Struktureinheiten der Formel (P7) die Gruppe B der ganz oder teilweise neutralisiert vorliegt. Daher enthalten derartige erfindungsgemäße kosmetische Mittel zusätzlich mindestens ein Alkanolamin. Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Erfindungsgemäße kosmetische Mittel, welche als festigendes anionisches Polymer mindestens ein Polymer enthalten, welches ausgewählt ist aus der Gruppe von
- Copolymeren aus Methacrylsäure und Ethylacrylat und tert-Butylacrylat (erhältlich unter den Handelsnamen Luvimer 100 P, Luvimer 30 E oder Luvimer 36 D von der Firma BASF SE),
- Copolymeren aus Acrylsäure und Ethylacrylat und tert-Butylacrylamid (erhältlich unter den Handelsnamen Ultrahold Strong oder Ultrahold 8 von der Firma BASF SE),
- Copolymeren aus 2-Acrylamido-2-methyl-propansulfonsäure und Acrylamid,
- Copolymeren aus 2-Acrylamido-2-methyl-propansulfonsäure, Acrylamid und Acrylsäure,
- Copolymeren von Acrylamid mit Methacrylsäure und Acryloyldimethyltaurat (erhältlich unter dem Handelsnamen Acudyne® SCP von der Firma Rohm & Haas),
- Copolymeren aus Vinylacetat und Crotonsäure (erhältlich unter dem Handelsnamen Aristoflex A 60 von der Firma Clariant),
- Copolymeren aus Vinylpropionat und Crotonsäure (erhältlich unter dem Handelsnamen Luviset CA 66 von der Firma BASF SE),
- Copolymeren aus Vinylneodecanoat, Vinylacetat und Crotonsäure (erhältlich unter den Handelsnamen Resyn 28-2942 bzw. Resyn 28-2930 von der Firma National Starch),
sind ganz besonders bevorzugt.

Weiterhin kann es erfindungsgemäß vorgesehen sein, das dass erfindungsgemäße Mittel als festigendes Polymer mindestens ein amphoteres festigendes Polymer enthält.

Bevorzugte amphotere festigende Polymere weisen mindestens eine Struktureinheit der Formel (P10) und mindestens eine Struktureinheit der Formel (P11) auf, 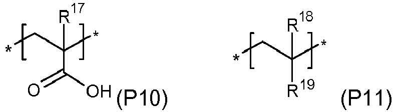 worin
R¹⁸ und R¹⁹, unabhängig voneinander, für ein Wasserstoffatom oder eine Methylgruppe stehen,
R¹⁷ für eine lineare oder verzweigte (C₄ bis C₁₂)-Alkylaminoethyl-aminocarbonylgruppe, eine lineare oder verzweigte (C₄ bis C₁₂)-Alkylaminopropylaminocarbonylgruppe, eine lineare oder verzweigte (C₄ bis C₁₂)-Alkylaminoethyloxycarbonylgruppe, eine lineare oder verzweigte (C₄ bis C₁₂)-Alkylaminopropyloxycarbonylgruppe steht.

Dabei ist es besonders bevorzugt, wenn die Struktureinheit der Formel (P10) ganz oder teilweise neutralisiert vorliegt. Daher enthalten derartige erfindungsgemäße kosmetische Mittel zusätzlich mindestens ein Alkanolamin. Geeignete Alkalsierungsmittel sind die zuvor im Zusammenhang mit anionischen festigenden Polymeren aufgeführten Alkalsierungsmittel. Vorzugsweise beträgt die in den erfindungsgemäßen kosmetischen Mitteln eingesetzte Menge an Alkalisierungsmittel 80 bis 100%, vorzugsweise 90 bis 100%, insbesondere 95 bis 100% der zur vollständigen Neutralisation der zusätzlichen amphoteren festigenden Polymere benötigten Menge.

Besonders geeignet ist mindestens ein amphoteres festigendes Polymer, welches neben jeweils mindestens einer der obigen Struktureinheiten der Formeln (P10) und (P11) zusätzlich mindestens eine Struktureinheit der Formel (P12) enthält 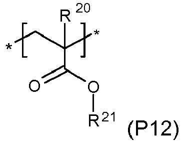 worin
R²⁰ für ein Wasserstoffatom oder eine Methylgruppe steht, und
R²¹ für eine (C₁ bis C₄)-Alkylgruppe, insbesondere eine Methylgruppe oder eine Ethylgruppe, steht.

Bevorzugte amphotere festigende Polymere dieser Art werden ausgewählt aus der Gruppe, die gebildet wird aus Copolymeren aus Acrylsäure, mindestens einem (C₁ bis C₄)-Alkylacrylat, mindestens einem C₄-Alkylaminoethylmethacrylat und mindestens einem C₈-Alkylacrylamid.

Die zusätzlichen filmbildenden und/oder festigenden Polymere können in den erfindungsgemäßen kosmetischen Mitteln bevorzugt in einer Menge von 0,1 Gew.-% bis 12,0 Gew.-%, vorzugsweise von 0,2 Gew.-% bis 10,0 Gew.-%, insbesondere von 0,5 Gew.-% bis 8,0 Gew.-%, jeweils bezogen auf das Gesamtgewicht des kosmetischen Mittels, enthalten sein.

Zur Intensivierung des erfindungsgemäßen Effektes enthalten die erfindungsgemäßen kosmetischen Mittel vorzugsweise zusätzlich mindestens ein Tensid. Tenside im Sinne der vorliegenden Erfindung sind amphiphile (bifunktionelle) Verbindungen, welche aus mindestens einem hydrophoben und mindestens einem hydrophilen Molekülteil bestehen. Der hydrophobe Rest ist bevorzugt eine Kohlenwasserstoffkette mit 8 bis 28 Kohlenstoff-Atomen, welche gesättigt oder ungesättigt, linear oder verzweigt sein kann. Besonders bevorzugt ist diese C₈-C₂₈-Alkylkette linear. Der hydrophile Molekülteil ist bevorzugt eine Polyethylenoxid-Gruppe mit mindestens 2 EO-Einheiten oder eine Säuregruppe. Eine Basiseigenschaft von Tensiden und Emulgatoren ist die orientierte Absorption an Grenzflächen sowie die Aggregation zu Mizellen und die Ausbildung von lyotrophen Phasen.

Im Rahmen der vorliegenden Erfindung können die kosmetischen Mittel zusätzlich mindestens ein Tensid aus der Gruppe von nichtionischen Tenside, anionischen Tensiden, amphoteren Tensiden sowie deren Mischungen, bevorzugt nichtionische Tenside, enthalten. Die Gruppe der ampholytischen oder auch amphoteren Tenside umfasst zwitterionische Tenside und Ampholyte.

Es hat sich als besonders bevorzugt erwiesen, wenn die erfindungsgemäßen Mittel zusätzlich mindestens ein nichtionisches Tensid enthalten. Nichtionische Tenside enthalten als hydrophile Gruppe z.B. eine Polyolgruppe, eine Polyalkylenglykolethergruppe oder eine Kombination aus Polyol- und Polyglykolethergruppe.

Als besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Erfindungsgemäße kosmetische Mittel mit hervorragenden Eigenschaften werden erhalten, wenn sie als nichtionische Tenside C₁₂-C₃₀-Fettsäuremono- und - diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl in einer Menge von 0,1 bis 5 Gew.-%, vorzugsweise von 0,2 bis 3 Gew.-%, insbesondere von 0,2 bis 1 Gew.-%, bezogen auf das Gesamtgewicht des kosmetischen Mittels, enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 Kohlenstoffatomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R²²-O-(CH₂-CH₂O)ₓ-CH₂-COOH, in welcher R²² eine lineare Alkylgruppe mit 8 bis 30 Kohlenstoffatomen und x = 0 oder 1 bis 16 ist,
- Acylsarcoside mit 8 bis 24 Kohlenstoffatomen in der Acylgruppe,
- Acyltauride mit 8 bis 24 Kohlenstoffatomen in der Acylgruppe,
- Acylisethionate mit 8 bis 24 Kohlenstoffatomen in der Acylgruppe,
- Sulfobernsteinsäuremono- und -dialkylester mit 8 bis 24 Kohlenstoffatomen in der Alkylgruppe und Sulfobernsteinsäuremono-alkylpolyoxyethylester mit 8 bis 24 Kohlenstoffatomen in der Alkylgruppe und 1 bis 6 Oxyethylgruppen,
- lineare Alkansulfonate mit 8 bis 24 Kohlenstoffatomen,
- lineare Alpha-Olefinsulfonate mit 8 bis 24 Kohlenstoffatomen,
- Alpha-Sulfofettsäuremethylester von Fettsäuren mit 8 bis 30 Kohlenstoffatomen,
- Alkylsulfate und Alkylpolyglykolethersulfate der Formel R²³-O(CH₂-CH₂O)ₓ-OSO₃H, in welcher R²³ eine bevorzugt lineare Alkylgruppe mit 8 bis 30 Kohlenstoffatomen und x = 0 oder 1 bis 12 ist,
- sulfatierte Hydroxyalkylpolyethylen- und/oder Hydroxyalkylenpropylenglykolether,
- Sulfonate ungesättigter Fettsäuren mit 8 bis 24 Kohlenstoffatomen und 1 bis 6 Doppelbindungen,
- sulfatierte Fettsäurealkylenglykolester der Formel R²⁴CO(AlkO)ₙSO₃M, in welcher R²⁴CO- für einen linearen oder verzweigten, aliphatischen, gesättigten und/oder ungesättigten Acylrest mit 6 bis 22 Kohlenstoffatomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren.

Erfindungsgemäß bevorzugte kosmetische Mittel dieser Ausführungsform enthalten das zusätzliche mindestens eine Tensid in einer Gesamtmenge von 0,01 Gew.-% bis 5 Gew.-%, insbesondere von 0,05 Gew.-% bis 0,5 Gew.-%, bezogen auf das Gesamtgewicht des kosmetischen Mittels.

Im Rahmen der vorliegenden Erfindung ist es bevorzugt, wenn die erfindungsgemäßen kosmetischen Mittel als Aerosolspray oder Nonaerosol-Spray konfektioniert werden. Als "Aerosolspray" wird ein kosmetisches Produkt verstanden, bei welchem das erfindungsgemäße kosmetische Mittel in einem druckfesten Behältnis unter Druck vorliegt und mittels Treibgas versprüht wird. Dahingegen liegen kosmetische Produkte in Form eines "Nonaerosol-Sprays" in einem unter Normaldruck stehendem Behältnis vor und werden mittels mechanischer Einwirkung durch ein Pump- oder Quetschsystem als Sprühnebel verteilt.

Liegt das erfindungsgemäße kosmetische Mittel als Aerosolspray vor, enthält dieses bevorzugt mindestens ein Treibmittel in einer Gesamtmenge von 10 bis 80 Gew.-%, vorzugsweise von 20 bis 70 Gew.-%, insbesondere von 30 bis 60 Gew.-%, bezogen auf das Gesamtgewicht des kosmetischen Mittels. Über das Mengenverhältnis von Treibmittel zu den übrigen Bestandteilen der kosmetischen Mittel lassen sich bei gegebener Sprühvorrichtung die Größen der Aerosoltröpfchen und die jeweilige Größenverteilung einstellen. Die Sprührate der erfindungsgemäßen Sprays beträgt bevorzugt 6,5 bis 10,0 g/10 s.

Erfindungsgemäß geeignete Treibmittel sind beispielsweise ausgewählt aus N₂O, Dimethylether, CO₂, Luft, Alkanen mit 3 bis 5 Kohlenstoffatomen, wie Propan, n-Butan, iso-Butan, n-Pentan und isoPentan, und deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treibmittel eingesetzt. Die vorliegende Erfindung umfasst aber ausdrücklich auch die Mitverwendung von Treibmitteln vom Typ der Fluorchlorkohlenwasserstoffe, insbesondere aber der Fluorkohlenwasserstoffe.

Das Treibmittel (insbesondere Dimethylether) ist in den erfindungsgemäßen Mitteln der Ausführungsform als Aerosolspray bevorzugt in einer Gesamtmenge von 30 bis 60 Gew.-%, bezogen auf das Gesamtgewicht des kosmetischen Mittels, enthalten.

Ganz besonders bevorzugt werden Dimethylether oder Mischungen von Propan und Butan als alleiniges Treibmittel im Gewichtsverhältnis Propan zu Butan von 20 zu 80 bis 15 zu 85 verwendet. Die Mischungen werden wiederum bevorzugt in den erfindungsgemäßen Mitteln in einer Gesamtmenge von 30 bis 55 Gew.-%, bezogen auf das Gesamtgewicht des kosmetischen Mittels, eingesetzt. Unter Butan wird erfindungsgemäß n-Butan, iso-Butan und Gemische aus n-Butan und iso-Butan verstanden. Am bevorzugtesten wird Dimethylether als alleiniges Treibmittel verwendet.

Falls die erfindungsgemäßen kosmetischen Mittel in Form eines "Nonaerosolsprays" vorliegen, können diese Mittel mit jedem beliebigen treibgasfreien Spraysystem, welches einen Spendebehälter und ein Sprühventil aufweist, ausgebracht werden, also z. B. in einer flexiblen Druckflasche mit Tauchrohr und Sprühventil (Squeeze Bottle), in einem Ballonzerstäuber, der nach dem Venturi-Prinzip arbeitet oder in einer Pumpen-Sprühflasche, deren Pumpenhebel mit dem Zeigefinger oder mit der ganzen Hand in der Art eines Abzugsbügels betätigt wird. In einer für die kosmetische Anwendung bevorzugten Ausführung weist der Spendebehälter eine manuell betätigte Sprühpumpe auf.

Die Struktureinheiten der Formeln (Ia), (IIa), (IIIa), (IVa), (Va) werden nach der CAS-Nomenklatur als Adipinsäure, 1,6-Hexandiol, Nepentylglycol, Isophorondicyanat, Isophorondiamin und N-(2-Aminoethyl)-3-aminoethannatriumsulfonat beschrieben. Weiterhin werden die Struktureinheiten der Formeln (VIa), (VIIa) sowie (VIIIa) nach der CAS-Nomenklatur als Polyethylen-polypropylenglycol-bis(2-methyl-2-propen-1-yl)ether, 3-[(2-Aminoethyl)amino]propyl-siloxan und Dimetyhlsiloxan beschrieben. Ein weiterer Gegenstand der vorliegenden Erfindung ist daher ein kosmetisches Mittel zur temporären Verformung keratinischer Fasern, enthaltend in einem kosmetisch verträglichen Träger
a) mindestens ein anionisches Polyurethanpolymer aus Adipinsäure, 1,6-Hexandiol, Nepentylglycol, Isophorondicyanat, Isophorondiamin und dem Natriumsalz der N-(2-Aminoethyl)-3-aminoethansulfonsäure, und
b) mindestens ein siliconhaltiges kationischen Copolymer aus Polyethylen-polypropylenglycol-bis(2-methyl-2-propen-1-yl)ether, 3-[(2-Aminoethyl)amino]propyl-siloxan und Dimetyhlsiloxan.

Überraschenderweise kommt es bei der zuvor genannten Kombination des speziellen anionischen Polyurethanpolymers und des speziellen siliconhaltigen kationischen Copolymers nicht zu einer negativen Wechselwirkung, so dass die erfindungsgemäßen kosmetischen Mittel sowohl die hevorragenden Glättungseigenschaften des anionischen Polyurethanpolymers als auch die hervorragenden Schutzeigenschaften vor Haarschädigung des kationischen siliconhaltigen Copolymers aufweisen. Die exzellente Kompatiblität dieser beiden speziellen Polymere resultiert darüber hinaus in äußerst lagerstabilen erfindungsgemäßen kosmetischen Mitteln.

Bezüglich weiterer bevorzugter Ausführungsformen gilt mutatis mutandis das zu den erfindungsgemäßen kosmetischen Mitteln Gesagte.

Darüber hinaus ist ein weiterer Gegenstand der vorliegenden Erfindung ein Verfahren zur temporären Verformung keratinischer Fasern, wobei das Verfahren die folgenden Verfahrensschritte umfasst:
a) Bereitstellen eines erfindungsgemäßen kosmetischen Mittels,
b) Applizieren des erfindungsgemäßen kosmetischen Mittels auf die keratinischen Fasern,
c) Verteilen des applizierten erfindungsgemäßen kosmetischen Mittels auf den keratinischen Fasern, und
d) Verformung der keratinischen Fasern bei einer Temperatur von 30 bis 250 °C in die gewünschte Form.

Im Rahmen des erfindungsgemäßen Verfahrens kann es vorgesehen sein, dass die Verformung der keratinischen Fasern bei einer Temperatur von 40 bis 250 °C, vorzugsweise von 50 bis 250 °C, bevorzugt von 80 bis 250 °C, insbesondere von 120 bis 250 °C, durchgeführt wird. Es ist erfindungsgemäß bevorzugt, wenn die Verformung unter Einsatz eines Heißluftföns oder eines Glätteines, insbesondere eines Glätteisens, durchgeführt wird. Die Erwärmung der keratinischen Fasern kann hierbei während der Verformung stattfinden. Es kann jedoch auch vorgesehen sein, die keratinischen Fasern nach der Applikation und/oder nach dem Verteilen des erfindungsgemäßen kosmetischen Mittels zu erwärmen.

Das erfindungsgemäße Verfahren resultiert in einem hervorragenden Glättungsergebnis, ohne jedoch aufgrund der bei der Glättung der keratinischen Fasern erforderlichen hohen Temperaturen zu einer Haarschädigung zu führen. Die mit dem erfindungsgemäßen Verfahren geglätten Haare weisen einen hervorragenden und langanhaltenden Frisurenhalt und eine hervorragende Nass- und/oder Trockenkämmbarkeit bzw. Pflege auf.

Bezüglich weiterer bevorzugter Ausführungsformen des erfindungsgemäßen Verfahrens gilt mutatis mutandis das zu den erfindungsgemäßen kosmetischen Mitteln Gesagte.

Schließlich ist ein weiterer Gegenstand der vorliegenden Erfindung die Verwendung eines erfindungsgemäßen kosmetischen Mittels zur temporären Verformung keratinischer Fasern bei einer Temperatur von 30 bis 250 °C.

Gemäß einer bevorzugten Ausführungsform dieses Gegenstands wird für die temporäre Verformung der keratinischen Fasern eine Temperatur von 40 bis 250 °C, vorzugsweise von 50 bis 250 °C, bevorzugt von 80 bis 250 °C, insbesondere von 120 bis 250 °C, verwendet. Die nachfolgenden Beispiele erläutern die vorliegende Erfindung, ohne sie jedoch darauf einzuschränken:

### Beispiele:

### 1. Formulierungen (alle Angaben in Gewichtsprozent, bezogen auf das Gesamtgewicht des jeweiligen kosmetischen Mittels):

| Rohstoff | E1 | E2 | E3 | E4 |
|---|---|---|---|---|
| Anionisches Polyurethanpolymer ¹⁾ | 0,5* | 1,0* | 1,2* | 1,5* |
| Kationisches siliconhaltiges Copolymer ²⁾ | 0,2* | 0,2* | 0,5* | 0,5* |
| Anionisches Copolymer ³⁾ | 0,2* | -- | 0,1* | -- |
| Ethanol | 5,0 | 5,0 | 5,0 | 5,0 |
| Propylenglycol | 1,0 | 1,0 | 1,0 | 1,0 |
| PEG-40 hydrogenated Castor Oil | 0,5 | 0,5 | 0,5 | 0,5 |
| Parfum | 0,15 | 0,15 | 0,15 | 0,15 |
| Milchsäure | Ad pH 4,5 | Ad pH 4,5 | Ad pH 4,5 | Ad pH 4,5 |
| Wasser | ad 100 | ad 100 | ad 100 | ad 100 |

| | | | | |
|---|---|---|---|---|
| ¹⁾ enthaltend Struktureinheiten der Formeln (Ia) bis (Va) mit a = 4, b = 6 und X⁺ = Na; INCI-Bezeichnung: Polyurethane-48 ²⁾ enthaltend Struktureinheiten der Formeln (VIa) bis (VIIIa) mit c = 20 und d = 35; INCI-Bezeichnung: Bis-Isobutyl PEG/PPG-20/35/Amodimethicone Copolymer ³⁾ INCI-Bezeichnung: Sodium Laneth-40 Maleate/Styrene Sulfonate Copolymer * Aktivsubstanzgehalt | | | | |

Die kosmetischen Mittel E1 bis E4 wurden durch Vermischen der obigen Inhaltsstoffe erhalten.

Anschließend wurden die kosmetischen Mittel auf keratinische Fasern appliziert und diese Fasern unter Verwendung eines Glätteisens bei Temperaturen zwischen 150 °C und 240 °C geglättet. Alle kosmetischen Mittel führten zu einer hervorragenden Glättwirkung und einem wirksamen Schutz vor Schädigungen der keratinischen Fasern aufgrund der hohen Temperaturen bzw zu einer guten Pflege, insbesondere Nass- und/oder Trockenkämmbarkeit der keratinischen Fasern.

Die Haarschädigung nach Glättung unter Verwendung eines erfindungsgemäßen kosmetischen Mittels E5 und einer Vergleichszusammensetzung V1 wurde durch Ermittlung des Haarbruchs nach der Glättung wie folgt bestimmt (Angaben in nachfolgender Tabelle in Gew.-%, bezogen auf das Gesamtgewicht der kosmetischen Mittel):

| Rohstoff | E5 | V1 |
|---|---|---|
| Anionisches Polyurethanpolymer ¹⁾ | 0,5* | -- |
| Kationisches siliconhaltiges Copolymer ²⁾ | 0,2* | -- |
| Ethanol | 5,0 | 5,0 |
| Propylenglycol | 0,5 | 0,5 |
| PEG-40 hydrogenated Castor Oil | 0,3 | 0,3 |
| Parfum | 0,2 | 0,2 |
| Milchsäure | Ad pH 4,5 | -- |
| Phosphorsäure 85%ig | -- | Ad pH 4,5 |
| Wasser | ad 100 | ad 100 |

| | | |
|---|---|---|
| ¹⁾ enthaltend Struktureinheiten der Formeln (Ia) bis (Va) mit a = 4, b = 6 und X⁺ = Na; INCI-Bezeichnung: Polyurethane-48 ²⁾ enthaltend Struktureinheiten der Formeln (VIa) bis (VIIIa) mit c = 20 und d = 35; INCI-Bezeichnung: Bis-Isobutyl PEG/PPG-20/35/Amodimethicone Copolymer | | |

Pro Zusammensetzung wurden jeweils 10 Haarsträhnen präpariert. Zunächst wurden die Haarsträhnen (IHIP - New York, European natural hair 6/0, lot #03/2013, S. 78, Länge: 12 cm, Gewicht: 1 ± 0.05 g) in einer 3 Gew.-%igen Lösung von Texapon NSO in Wasser für 5 Minuten in einem Ultraschallbad gereinigt und für 5 Minuten unter Kämmen mit Leitungswasser ausgewaschen. Nach Trocknung mit dem Handtuch wurden jeweils 0.5 g der jeweiligen Zusammensetzung pro Gramm Haarsträhne aufgetragen und einmassiert. Anschließend wurden die Haarsträhnen automatisch gekämmt (Kamm: Hercules Sägemann, feinzahniger Hartgummikamm) und gleichzeitig bei 110 °C für 5 Minuten trockengefönt. Alle Strähnen wurden bei 25 °C und 25 % relativer Luftfeuchtigkeit für mindestens 24 Stunden gelagert und anschließend auf 25 °C und 50 % relativer Luftfeuchtigkeit für mindestens 1 Stunde equilibriert und gewogen.

Jede Strähne wurde unter konstanten Umgebungsbedingungen (25°C, 50 % relative Luftfeuchtigkeit) 20.000 mal gekämmt und der Haarbruch in einer Schachtel unter dem Kamm gesammelt. Die Menge an abgebrochenem Haar wurde ausgewogen und in Relation zu dem Gewicht der jeweiligen Haarsträhne vor dem Kämmen gesetzt. Alle Ergebnisse wurden mittels statistischer Tests (Shapiro-Wilks Test, Bartlett Test, Newman-Keuls Test) überprüft und waren signifikant. Es wurden die folgenden Ergebnisse erhalten:

| Zusammensetzung | Haarbruch [%] |
|---|---|
| V1 | 8,7 |
| E1 | 4,6 |

Der Einsatz der erfindungsgemäßen Kombination eines anionischen Polyurethanpolymers und eines siliconhaltigen kationischen Copolymers führt während der Haarglättung zu einem signifikant verminderten Haarbruch und somit zu einer signifikant verminderten Haarschädigung.

## Patentansprüche

1. Kosmetisches Mittel zur temporären Verformung keratinischer Fasern, enthaltend in einem kosmetisch verträglichen Träger
a) mindestens ein anionisches Polyurethanpolymer, umfassend mindestens eine Struktureinheit der Formel (I) und mindestens eine Struktureinheit der Formel (II) und mindestens eine Struktureinheit der Formel (III) und mindestens eine Struktureinheit der Formel (IV) und mindestens eine Struktureinheit der Formel (V) 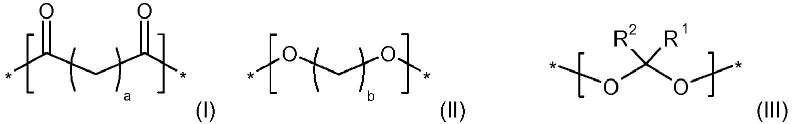 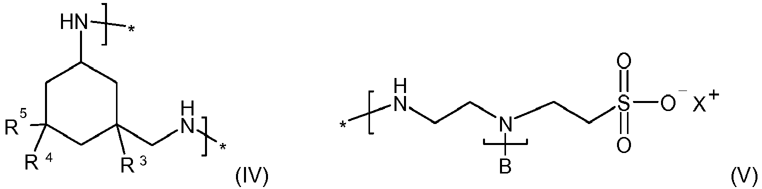 worin
a und b, unabhängig voneinander, für ganze Zahlen von 1 bis 20 stehen,
R¹, R², R³, R⁴ und R⁵, jeweils unabhängig voneinander, für ein Wasserstoffatom oder für eine C₁-C₄-Alkylgruppe stehen,
B für ein Wasserstoffatom oder eine direkte Bindung zu einer weiteren Struktureinheit steht,
X⁺ für ein physiologisch verträgliches Kation steht, und
b) mindestens ein siliconhaltiges kationisches Copolymer, umfassend mindestens eine Struktureinheit der Formel (VI) und mindestens eine Struktureinheit der Formel (VII) und mindestens eine Struktureinheit der Formel (VIII) 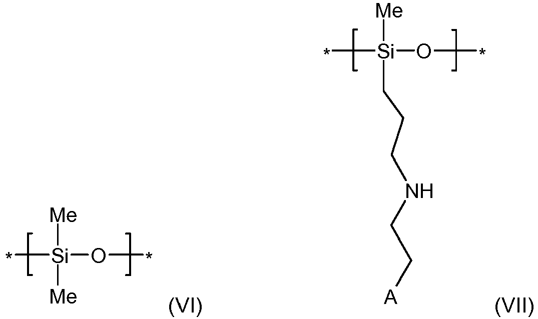 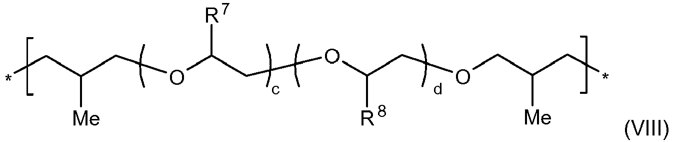 worin
A für eine Hydroxylgruppe, eine Aminogruppe oder eine Thiolgruppe steht,
R⁷ und R⁸, jeweils unabhängig voneinander, für ein Wasserstoffatom, eine Methylgruppe, eine Ethylgruppe, eine C₁-C₄-Aralkylgruppe, eine C₂-C₆-Alkenylgruppe oder eine C₂-C₆-Hydroxyalkylgruppe stehen, und
c und d, jeweils unabhängig voneinander, für ganze Zahlen von 10 bis 55 stehen.

2. Kosmetisches Mittel nach Anspruch 1, **dadurch gekennzeichnet, dass** in der Struktureinheit der Formel (I) a für ganze Zahlen von 1 bis 10, vorzugsweise von 2 bis 8, insbesondere von 2 bis 6, steht und dass in der Struktureinheit der Formel (II) b für ganze Zahlen von 1 bis 12, vorzugsweise von 2 bis 10, insbesondere von 4 bis 8, steht.

3. Kosmetisches Mittel nach einem der Ansprüche 1 oder 2, **dadurch gekennzeichnet, dass** das mindestens eine anionische Polyurethanpolymer a) mindestens eine Struktureinheit der Formel (la) und mindestens eine Struktureinheit der Formel (IIa) und mindestens eine Struktureinheit der Formel (lila) und mindestens eine Struktureinheit der Formel (IVa) und mindestens eine Struktureinheit der Formel (Va) enthält 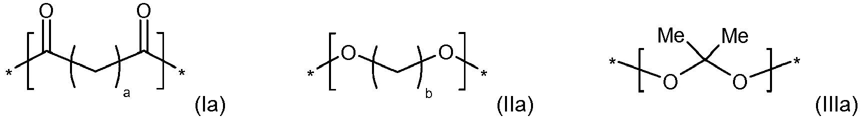 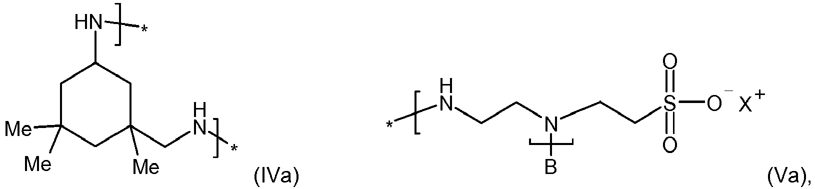 worin
a für ganze Zahlen von 2 bis 6 steht,
b für ganze Zahlen von 4 bis 8 steht, und
X⁺ für ein physiologisch verträgliches Kation, insbesondere Natrium, steht.

4. Kosmetisches Mittel nach einem der vorangehenden Ansprüche, **dadurch gekennzeichnet, dass** das mindestens eine anionische Polyurethanpolymer a) in einer Gesamtmenge von 0,05 bis 5 Gew.-%, vorzugsweise von 0,08 bis 3 Gew.-%, bevorzugt von 0,1 bis 2 Gew.-%, insbesondere von 0,2 bis 1,5 Gew.-%, bezogen auf das Gesamtgewicht des kosmetischen Mittels, enthalten ist.

5. Kosmetisches Mittel nach einem der vorangehenden Ansprüche, **dadurch gekennzeichnet, dass** in der Struktureinheit der Formel (VII) der Rest A für eine Aminogruppe steht.

6. Kosmetisches Mittel nach einem vorangehenden Ansprüche, **dadurch gekennzeichnet, dass** in der Struktureinheit der Formel (VIII) die Reste R⁷ und R⁸, jeweils unabhängig voneinander, für ein Wasserstoffatom oder eine Methylgruppe, bevorzugt R⁷ für Wasserstoff und R⁸ für eine Methylgruppe, stehen.

7. Kosmetisches Mittel nach einem der vorangehenden Ansprüche, **dadurch gekennzeichnet, dass** in der Struktureinheit der Formel (VIII) c für ganze Zahlen von 15 bis 30, insbesondere von 15 bis 25, steht und d für ganze Zahlen von 20 bis 45, bevorzugt von 25 bis 40, steht.

8. Kosmetisches Mittel nach einem der vorangehenden Ansprüche, **dadurch gekennzeichnet, dass** das mindestens eine siliconhaltige kationische Copolymer b) mindestens eine Struktureinheit der Formel (VIa) und mindestens eine Struktureinheit der Formel (Vlla) und mindestens eine Struktureinheit der Formel (VIIIa) enthält 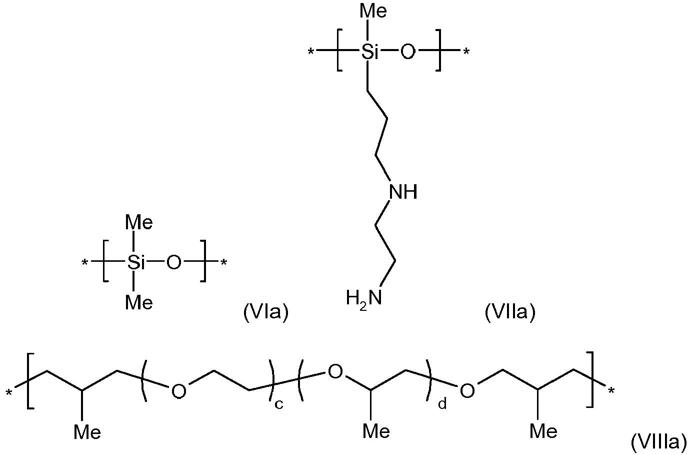 worin
c für ganze Zahlen von 15 bis 25 steht, und
d für ganze Zahlen von 25 bis 40 steht.

9. Kosmetisches Mittel nach einem der vorangehenden Ansprüche, **dadurch gekennzeichnet, dass** das mindestens eine siliconhaltiges Copolymer b) in einer Gesamtmenge von 0,01 bis 2 Gew.-%, vorzugsweise von 0,02 bis 1,5 Gew.-%, bevorzugt 0,03 bis 1 Gew.-%, insbesondere 0,05 bis 0,5 Gew.-%, bezogen auf das Gesamtgewicht des kosmetischen Mittels, enthalten ist.

10. Kosmetisches Mittel nach einem der vorangehenden Ansprüche, **dadurch gekennzeichnet, dass** es
a) mindestens ein anionisches Polyurethanpolymer enthält, welches mindestens eine Struktureinheit der Formel (la) und mindestens eine Struktureinheit der Formel (IIa) und mindestens eine Struktureinheit der Formel (lila) und mindestens eine Struktureinheit der Formel (IVa) und mindestens eine Struktureinheit der Formel (Va) aufweist 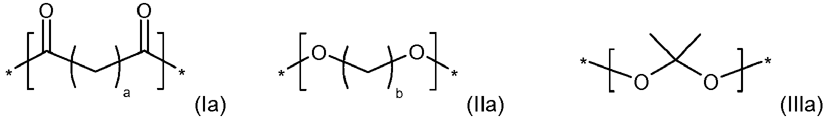 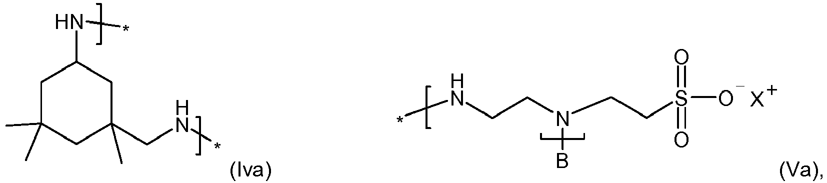 worin
a für ganze Zahlen von 2 bis 6 steht,
b für ganze Zahlen von 4 bis 8 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht, und
b) mindestens ein kationisches siliconhaltiges Copolymer enthält, welches mindestens eine mindestens eine Struktureinheit der Formel (VIa), mindestens eine Struktureinheit der Formel (Vlla) und mindestens eine Struktureinheit der Formel (VIIIa) enthält 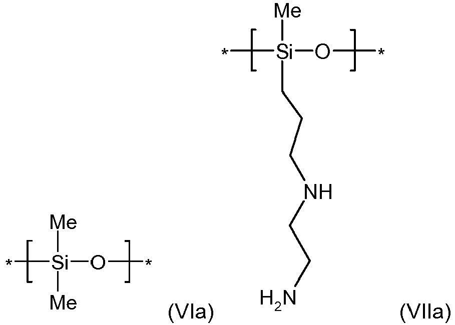 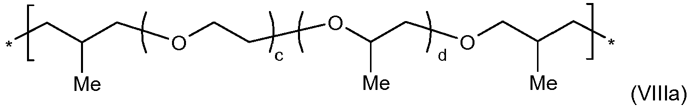 worin
c für ganze Zahlen von 15 bis 25 steht, und
d für ganze Zahlen von 25 bis 40 steht.

11. Kosmetisches Mittel nach einem der vorangehenden Ansprüche, **dadurch gekennzeichnet, dass** das kosmetische Mittel ein Gewichtsverhältnis der Gesamtmenge des mindestens einen anionischen Polyurethanpolymers a) zu der Gesamtmenge des mindestens einen siliconhaltigen Copolymers b) von 300 : 1 bis 1 : 40, vorzugsweise von 100 : 1 bis 1 : 20, bevorzugt von 40 : 1 bis 1 : 5, weiter bevorzugt von 20 : 1 bis 1 : 1, insbesondere von 10 : 1 bis 2 : 1, aufweist.

12. Kosmetisches Mittel nach einem der vorangehenden Ansprüche, **dadurch gekennzeichnet, dass** das kosmetische Mittel zusätzlich ein anionisches Polymer, umfassend mindestens eine Struktureinheit der Formel (IX) und mindestens eine Struktureinheit der Formel (X) 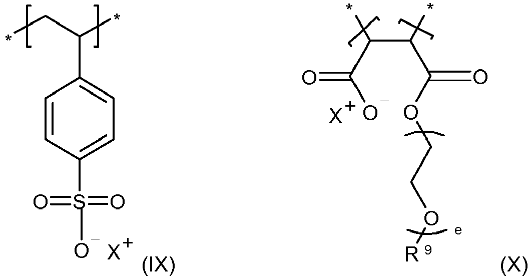 worin
R⁹ für eine lineare oder verzweigte, gesättigte oder ungesättigte C₁₀-C₄₀-Alkylgruppe oder eine C₁₀-C₄₀-Hydroxyalkylgruppe, insbesondere für eine lineare oder verzweigte, gesättigte oder ungesättigte C₁₄-C₃₆-Alkylgruppe, steht,
e für ganze Zahlen zwischen 20 und 70, vorzugsweise 30 und 60, insbesondere 35 und 45, steht, und
X⁺ für ein physiologisch verträgliches Kation steht,
enthält.

13. Kosmetisches Mittel zur temporären Verformung keratinischer Fasern, enthaltend in einem kosmetisch verträglichen Träger
a) mindestens ein anionisches Polyurethanpolymer aus Adipinsäure, 1,6-Hexandiol, Nepentylglycol, Isophorondicyanat, Isophorondiamin und dem Natriumsalz der N-(2-Aminoethyl)-3-aminoethansulfonsäure, und
b) mindestens ein siliconhaltiges kationisches Copolymer aus Polyethylen-polypropylenglycol-bis(2-methyl-2-propen-1-yl)ether, 3-[(2-Aminoethyl)amino]propyl-siloxan und Dimetyhlsiloxan.

14. Verfahren zur temporären Verformung keratinischer Fasern, wobei das Verfahren die folgenden Verfahrensschritte umfasst:
a) Bereitstellen eines kosmetischen Mittels nach einem der Ansprüche 1 bis 13,
b) Applizieren des kosmetischen Mittels nach einem der Ansprüche 1 bis 13 auf die keratinischen Fasern,
c) Verteilen des applizierten kosmetischen Mittels nach einem der Ansprüche 1 bis 13 auf den keratinischen Fasern, und
d) Verformung der keratinischen Fasern bei einer Temperatur von 30 bis 250 °C in die gewünschte Form.

15. Verwendung eines kosmetischen Mittels gemäß einem der Ansprüche 1 bis 13 zur temporären Verformung keratinischer Fasern bei einer Temperatur von 30 bis 250 °C.

## Claims

1. A cosmetic agent for temporarily shaping keratin fibers, containing, in a cosmetically acceptable carrier,
a) at least one anionic polyurethane polymer, comprising at least one structural unit of formula (I), at least one structural unit of formula (II), at least one structural unit of formula (III), at least one structural unit of formula (IV) and at least one structural unit of formula (V), 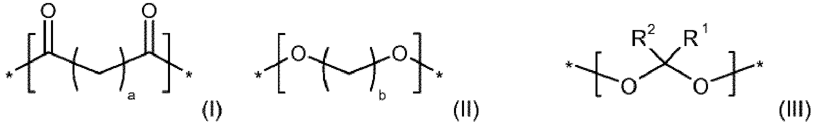 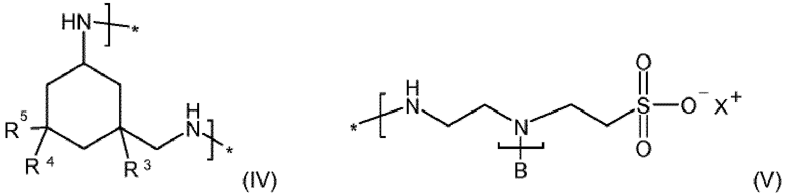 in which
a and b represent, independently of one another, integers from 1 to 20,
R¹, R², R³, R⁴ and R⁵ represent, in each case independently of one another, a hydrogen atom or a C₁-C₄ alkyl group,
B represents a hydrogen atom or a direct bond to another structural unit,
X⁺ represents a physiologically acceptable cation, and
b) at least one silicone-containing cationic copolymer comprising at least one structural unit of formula (VI), at least one structural unit of formula (VII) and at least one structural unit of formula (VIII), 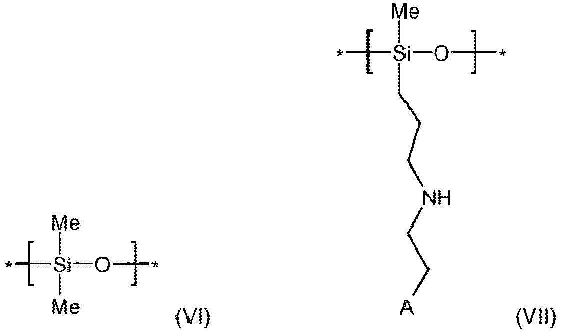 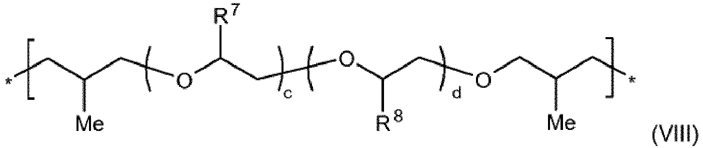 in which
A represents a hydroxyl group, an amino group, or a thiol group,
R⁷ and R⁸ represent, in each case independently of one another, a hydrogen atom, a methyl group, an ethyl group, a C₁-C₄ aralkyl group, a C₂-C₆ alkenyl group or a C₂-C₆ hydroxyalkyl group, and
c and d represent, in each case independently of one another, integers from 10 to 55.

2. The cosmetic agent according to claim 1, **characterized in that**, in the structural unit of formula (I), a represents integers from 1 to 10, preferably 2 to 8, in particular 2 to 6, and **in that**, in the structural unit of formula (II), b represents integers from 1 to 12, preferably 2 to 10, in particular 4 to 8.

3. The cosmetic agent according to one of claims 1 or 2, **characterized in that** the at least one anionic polyurethane polymer a) contains at least one structural unit of formula (la), at least one structural unit of formula (IIa), at least one structural unit of formula (IIIa), at least one structural unit of formula (IVa) and at least one structural unit of formula (Va) 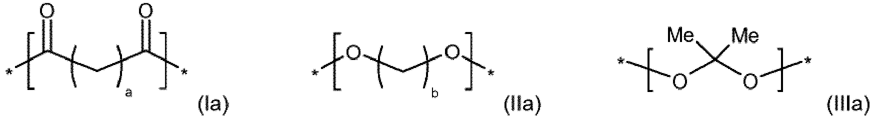 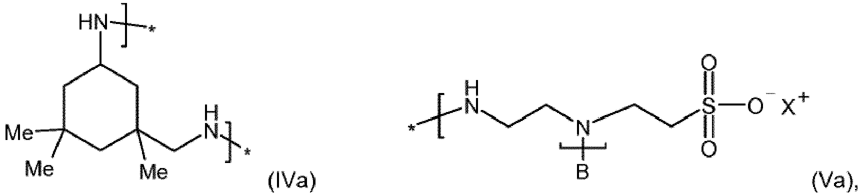 in which
a represents integers from 2 to 6,
b represents integers from 4 to 8, and
X+ represents a physiologically acceptable cation, in particular sodium.

4. The cosmetic agent according to one of the preceding claims, **characterized in that** the at least one anionic polyurethane polymer a) is contained in a total amount of from 0.05 to 5 wt.%, preferably 0.08 to 3 wt.%, more preferably 0.1 to 2 wt.%, in particular 0.2 to 1.5 wt.%, based on the total weight of the cosmetic agent.

5. The cosmetic agent according to one of the preceding claims, **characterized in that**, in the structural unit of formula (VII), the functional group A represents an amino group.

6. The cosmetic agent according to one of the preceding claims, **characterized in that**, in the structural unit of formula (VIII), the functional groups R⁷ and R⁸ represent, in each case independently of one another, a hydrogen atom or a methyl group, preferably R⁷ represents hydrogen and R⁸ represents a methyl group.

7. The cosmetic agent according to one of the preceding claims, **characterized in that**, in the structural unit of formula (VIII), c represents integers from 15 to 30, in particular 15 to 25, and d represents integers from 20 to 45, preferably 25 to 40.

8. The cosmetic agent according to one of the preceding claims, **characterized in that** the at least one silicone-containing cationic copolymer b) contains at least one structural unit of formula (VIa), at least one structural unit of formula (VIIa) and at least one structural unit of formula (VIIIa) 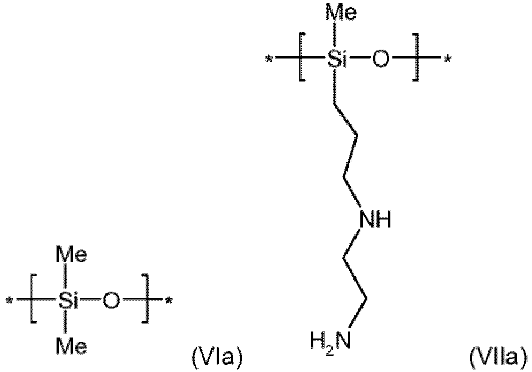 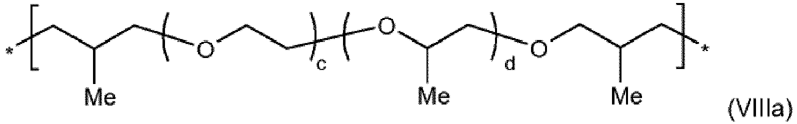 in which
c represents integers from 15 to 25 and
d represents integers from 25 to 40.

9. The cosmetic agent according to one of the preceding claims, **characterized in that** the at least one silicone-containing copolymer b) is contained in a total amount of from 0.01 to 2 wt.%, preferably 0.02 to 1.5 wt.%, more preferably 0.03 to 1 wt.%, in particular 0.05 to 0.5 wt.%, based on the total weight of the cosmetic agent.

10. The cosmetic agent according to one of the preceding claims, **characterized in that** it contains
a) at least one anionic polyurethane polymer, which has at least one structural unit of formula (la), at least one structural unit of formula (IIa), at least one structural unit of formula (IIIa), at least one structural unit of formula (IVa) and at least one structural unit of formula (Va) 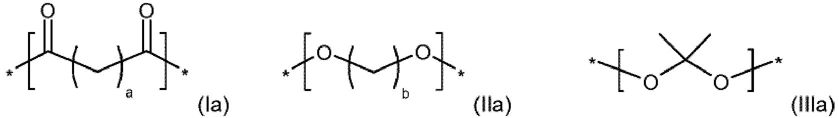 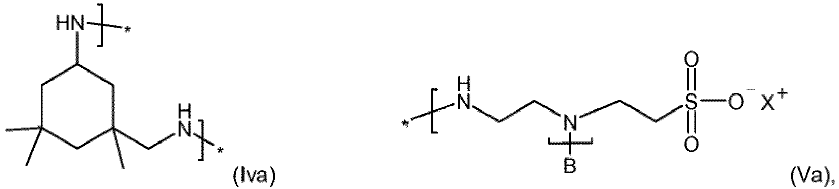 in which
a represents integers from 2 to 6,
b represents integers from 4 to 8, and
X+ represents metal cations of the physiologically acceptable metals from groups la, Ib, IIa, IIb, IIIb, VIa, or VIII of the periodic table of the elements, ammonium ions, as well as cationic organic compounds having a quaternized nitrogen atom, and
b) at least one cationic silicone-containing copolymer which contains at least one structural unit of formula (VIa), at least one structural unit of formula (VIIa) and at least one structural unit of formula (VIIIa), 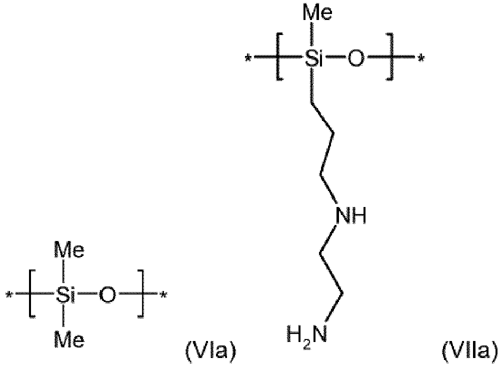 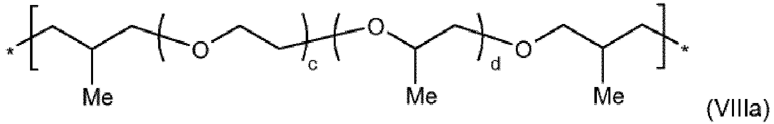 in which
c represents integers from 15 to 25 and
d represents integers from 25 to 40.

11. The cosmetic agent according to one of the preceding claims, **characterized in that** the cosmetic agent has a weight ratio of the total amount of the at least one anionic polyurethane polymer a) to the total amount of the at least one silicone-containing copolymer b) of from 300:1 to 1:40, preferably 100:1 to 1:20, more preferably 40:1 to 1:5, even more preferably 20:1 to 1:1, in particular 10:1 to 2:1.

12. The cosmetic agent according to one of the preceding claims, **characterized in that** the cosmetic agent additionally contains an anionic polymer, comprising at least one structural unit of formula (IX) and at least one structural unit of formula (X) 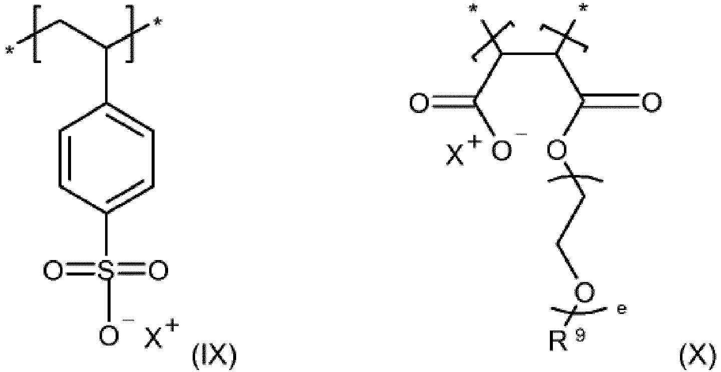 in which
R⁹ represents a linear or branched, saturated or unsaturated C₁₀-C₄₀ alkyl group or a C₁₀-C₄₀ hydroxyalkyl group, in particular a linear or branched, saturated or unsaturated C₁₄-C₃₆ alkyl group,
e represents integers between 20 and 70, preferably 30 and 60, in particular 35 and 45, and
X⁺ represents a physiologically acceptable cation.

13. The cosmetic agent for temporarily shaping keratin fibers, containing, in a cosmetically acceptable carrier,
a) at least one anionic polyurethane polymer of adipic acid, 1,6-hexanediol, neopentyl glycol, isophorone dicyanate, isophorone diamine and the sodium salt of N-(2-aminoethyl)-3-aminoethane sulfonic acid, and
b) at least one silicone-containing cationic copolymer of polyethylene-polypropylene glycol-bis(2-methyl-2-propene-1-yl) ether, 3-[(2-aminoethyl)amino]propyl-siloxane and dimethylsiloxane.

14. A method for temporarily deforming keratin fibers, wherein the method comprises the following method steps:
a) preparing a cosmetic agent according to one of claims 1 to 13;
b) applying the cosmetic agent according to one of claims 1 to 13 to the keratin fibers;
c) distributing the applied cosmetic agent according to one of claims 1 to 13 on the keratin fibers; and
d) shaping the keratin fibers into the desired shape at a temperature of from 30 °C to 250 °C.

15. The use of a cosmetic agent according to one of claims 1 to 13 for temporarily shaping keratin fibers at a temperature of from 30 °C to 250 °C.

## Revendications

1. Agent cosmétique, destiné à la mise en forme temporaire de fibres de kératine, contenant dans un support cosmétiquement acceptable
a) au moins un polymère de polyuréthane anionique comprenant au moins une unité structurelle de la formule (I) et au moins une unité structurelle de la formule (II) et au moins une unité structurelle de la formule (III) et au moins une unité structurelle de la formule (IV) et au moins une unité structurelle de la formule (V) 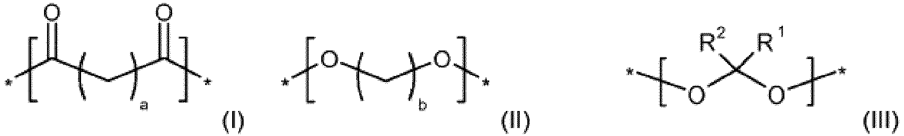 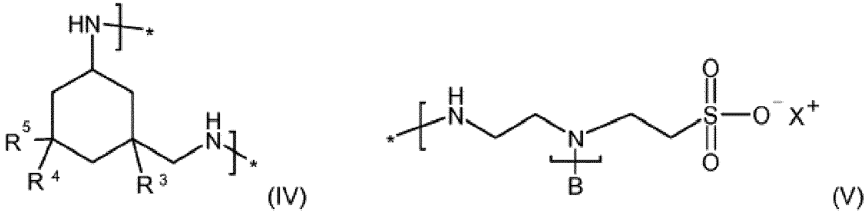 formules dans lesquelles
a et b sont indépendamment des nombres entiers de 1 à 20,
R¹, R², R³, R⁴ et R⁵ représentent chacun indépendamment un atome d'hydrogène ou un groupe alkyle en C₁ à C₄,
B représente un atome d'hydrogène ou une liaison directe à une autre unité structurelle,
X+ représente un cation physiologiquement acceptable, et
b) au moins un copolymère cationique silicié comprenant au moins une unité structurelle de la formule (VI) et au moins une unité structurelle de la formule (VII) et au moins une unité structurelle de la formule (VIII) 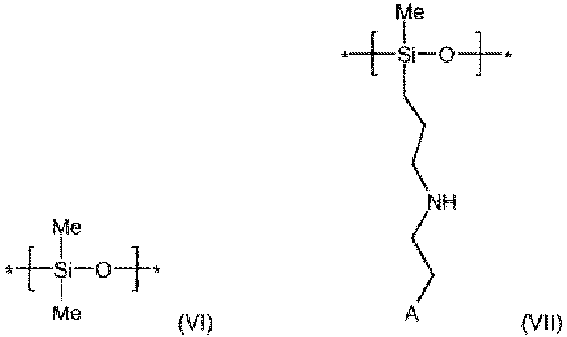 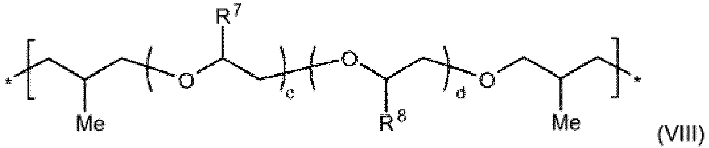 formules dans lesquelles
A représente un groupe hydroxyle, un groupe amino ou un groupe thiol,
R⁷ et R⁸ représentent chacun indépendamment un atome d'hydrogène, un groupe méthyle, un groupe éthyle, un groupe alkyle en C₁ à C₄, un groupe alcényle en C₂ à C₆ ou un groupe hydroxyalkyle en C₂ à C₆, et
c et d sont chacun indépendamment des nombres entiers de 10 à 55.

2. Agent cosmétique selon la revendication 1, **caractérisé en ce que**, dans l'unité structurelle de la formule (I), a est un nombre entier de 1 à 10, de préférence de 2 à 8, en particulier de 2 à 6, et **en ce que**, dans l'unité structurelle de la formule (II), b est un nombre entier de 1 à 12, de préférence de 2 à 10, en particulier de 4 à 8.

3. Agent cosmétique selon l'une des revendications 1 ou 2, **caractérisé en ce que** l'au moins un polymère de polyuréthane anionique a) contient au moins une unité structurelle de la formule (la) et au moins une unité structurelle de la formule (IIa) et au moins une unité structurelle de la formule (IIIa) et au moins une unité structurelle de la formule (IVa) et au moins une unité structurelle de la formule (Va) 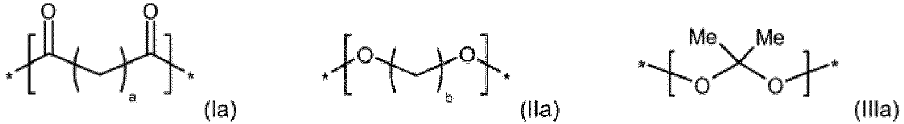 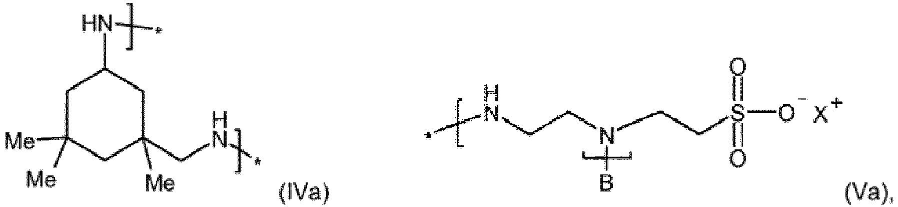 formules dans lesquelles
a est un nombre entier de 2 à 6,
b est un nombre entier de 4 à 8, et
X+ est un cation physiologiquement acceptable, en particulier du sodium.

4. Agent cosmétique selon l'une des revendications précédentes, **caractérisé en ce que** l'au moins un polymère de polyuréthane anionique a) est contenu dans une quantité totale de 0,05 à 5 % en poids, avantageusement de 0,08 à 3 % en poids, de préférence de 0,1 à 2 % en poids, en particulier de 0,2 à 1,5 % en poids, par rapport au poids total de l'agent cosmétique.

5. Agent cosmétique selon l'une des revendications précédentes, **caractérisé en ce que**, dans l'unité structurelle de la formule (VII), le radical A représente un groupe amino.

6. Agent cosmétique selon l'une des revendications précédentes, **caractérisé en ce que**, dans l'unité structurelle de la formule (VIII), les radicaux R⁷ et R⁸ représentent chacun indépendamment un atome d'hydrogène ou un groupe méthyle, de préférence R⁷ représente un atome d'hydrogène et R⁸ représente un groupe méthyle.

7. Agent cosmétique selon l'une des revendications précédentes, **caractérisé en ce que**, dans l'unité structurelle de la formule (VIII), c est un nombre entier de 15 à 30, en particulier de 15 à 25, et D est un nombre entier de 20 à 45, de préférence de 25 à 40.

8. Agent cosmétique selon l'une des revendications précédentes, **caractérisé en ce que** l'au moins un copolymère cationique silicié b) contient au moins une unité structurelle de la formule (VIa) et au moins une unité structurelle de la formule (VIIa) et au moins une unité structurelle de la formule (Villa) 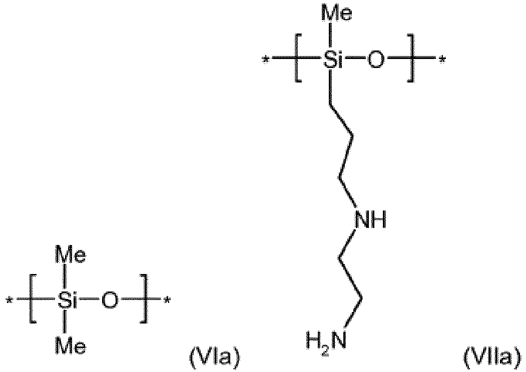 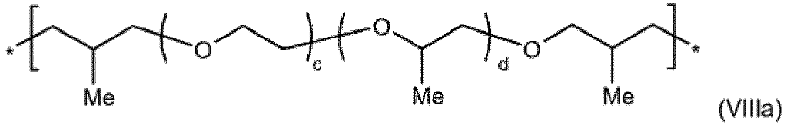 formules dans lesquelles
c est un nombre entier de 15 à 25, et
d est un nombre entier de 25 à 40.

9. Agent cosmétique selon l'une des revendications précédentes, **caractérisé en ce que** l'au moins un copolymère silicié b) est contenu dans une quantité totale de 0,01 à 2 % en poids, avantageusement de 0,02 à 1,5 % en poids, de préférence de 0,03 à 1 % en poids, en particulier de 0,05 à 0,5 % en poids, par rapport au poids total de l'agent cosmétique.

10. Agent cosmétique selon l'une des revendications précédentes, **caractérisé en ce qu'**il contient
a) au moins un polymère de polyuréthane anionique qui comporte au moins une unité structurelle de la formule (la) et au moins une unité structurelle de la formule (IIa) et au moins une unité structurelle de la formule (IIIa) et au moins une unité structurelle de la formule (IVa) et au moins une unité structurelle de la formule (Va) 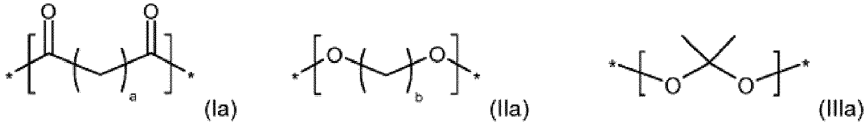 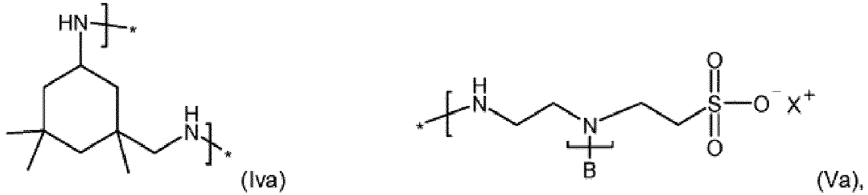 formules dans lesquelles
a est un nombre entier de 2 à 6.
b est un nombre entier de 4 à 8, et
X+ représente des cations métalliques de métaux physiologiquement acceptables des groupes la, Ib, IIa, IIb, IIIb, VIa ou VIII du Tableau Périodique des Eléments, des ions ammonium et des composés organiques cationiques ayant un atome d'azote quaternisé, et
b) au moins un copolymère cationique silicié qui contient au moins une unité structurelle de la formule (VIa), au moins une unité structurelle de la formule VIIa) et au moins une unité structurelle de la formule (Villa) 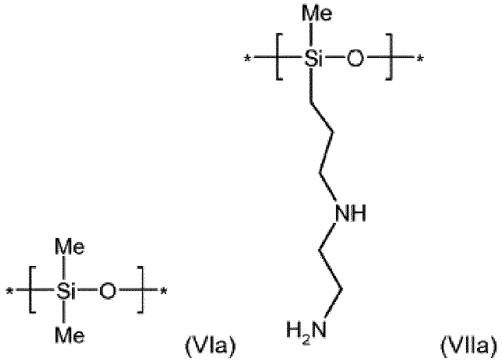 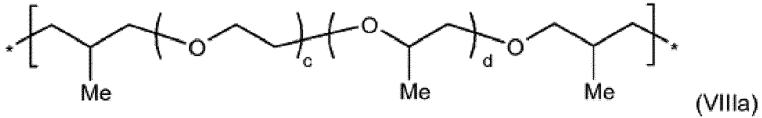 formules dans lesquelles
c est un nombre entier de 15 à 25, et
d est un nombre entier de 25 à 40.

11. Agent cosmétique selon l'une des revendications précédentes, **caractérisé en ce que** l'agent cosmétique a un rapport en poids de la quantité totale de l'au moins un polymère de polyuréthane anionique a) à la quantité totale de l'au moins un copolymère silicié b) de 300:1 à 1:40, de préférence de 100:1 à 1:20, de préférence de 40:1 à 1:5, plus préférablement de 20:1 à 1:1, en particulier de 10:1 à 2:1.

12. Agent cosmétique selon l'une des revendications précédentes, **caractérisé en ce que** l'agent cosmétique contient en outre un polymère anionique comportant au moins une unité structurelle de la formule (IX) et au moins une unité structurelle de la formule (X) 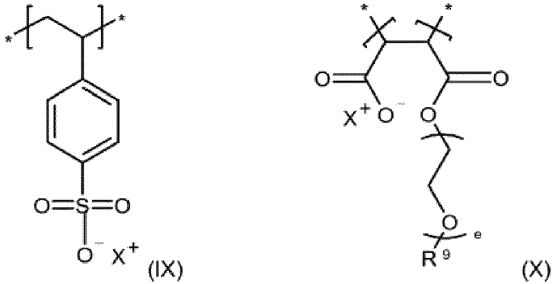 formules dans lesquelles
R⁹ représente un groupe alkyle en C₁₀ à C₄₀ linéaire ou ramifié, saturé ou insaturé, ou un groupe hydroxyalkyle en C₁₀ à C₄₀, en particulier un groupe alkyle en C₁₄ à C₃₆ linéaire ou ramifié, saturé ou insaturé,
e est un nombre entier compris entre 20 et 70, de préférence 30 et 60, en particulier 35 et 45, et
X+ est un cation physiologiquement acceptable.

13. Agent cosmétique destiné à la mise en forme temporaire de fibres de kératine, contenant dans un support cosmétiquement acceptable
a) au moins un polymère de polyuréthane anionique constitué d'acide adipique, de 1,6-hexanediol, de néopentylglycol, de diisocyanate d'isophorone, d'isophorone diamine et du sel de sodium de l'acide N-(2-aminoéthyl)-3-aminoéthansulfonique, et
b) au moins un copolymère cationique silicié constitué de polyéthylène-polypropylène glycol bis(2-méthyl-2-propèn-1-yl)éther, de 3-[(2-aminoéthyl)amino]propyl-siloxane et de diméthylsiloxane.

14. Procédé de mise en forme temporaire de fibres de kératine, le procédé comprenant les étapes suivantes :
a) fournir un agent cosmétique selon l'une des revendications 1 à 13,
b) appliquer l'agent cosmétique selon l'une des revendications 1 à 13 sur les fibres de kératine,
c) répartir l'agent cosmétique appliqué selon l'une des revendications 1 à 13 sur les fibres kératiniques, et
d) mettre en forme les fibres de kératine à une température de 30 à 250 °C selon la forme souhaitée.

15. Utilisation d'un agent cosmétique selon l'une des revendications 1 à 13 pour la mise en forme temporaire de fibres de kératine à une température de 30 à 250 °C.
